# EUROPEAN PATENT APPLICATION

(11) **EP 4 592 318 A1**
(43) Date of publication of application: **30.07.2025**
(21) Application number: 23881860.3
(22) Date of filing: 25.10.2023
(51) Int. Cl.: C07K 16/28, A61P 35/00, A61K 39/395

(54) **HUMANIZED L1CAM ANTIBODY-DRUG CONJUGATE**

(30) Priority: 26.10.2022 CN 202211317321
(71) Applicant: Curon Biopharmaceutical (Shanghai) Co., Limited, Shanghai 201309 (CN)
(72) Inventor: WANG, Ji, Shanghai 201309 (CN); DING, Zhilou, Shanghai 201309 (CN); CHEN, Zhihong, Shanghai 201309 (CN); GU, Jinming, Shanghai 201309 (CN); YANG, Qiumei, Shanghai 201309 (CN)
(74) Representative: E. Blum & Co. AG
(86) International application number: PCT/CN2023/126347
(87) International publication number: WO 2024/088283

(57) **Abstract**

Provided in the present invention are an anti-L1CAM antibody-drug conjugate, and a preparation method therefor and the pharmaceutical use thereof. In particular, provided in the present invention are an antibody-drug conjugate (ADC) containing an anti-L1CAM antibody conjugated to MMAE or a derivative thereof; a pharmaceutical composition containing the ADC; and the use thereof in the preparation of a drug for treating diseases or conditions mediated by L1CAM, particularly, the use thereof in the preparation of anti-cancer drugs.

## Description

### Cross Reference

This patent application is related to and claims the benefit of priority to Chinese Patent Application No.202211317321.5, filed on October 26, 2022, the content of which is incorporated by reference in its entirety.

### Technical Field

The invention relates to the field of biology and pharmaceutic, and particularly, relates to humanized anti-L1CAM antibody-drug conjugates.

### Background

L1 cell adhesion molecule (L1CAM), also known as CD171, is a transmembrane glycoprotein with a molecular weight ranging from 200,000 to 220,000 Daltons [Miriam van der Maten et al., Int. J. Mol. Sci., 2019, 20:4180*.*]*.* In heathy person, the expression of L1 cell adhesion molecule is mainly confined to the nervous system, with a small amount of expression in kidney tissue and skin *[*Annette Kunkele et al., Clin. Cancer Res., 2016, 23:466-477.]. L1 cell adhesion molecule is highly expressed in various tumors and is closely associated with the proliferation and drug resistance of multiple types of tumor cells.

L1 cell adhesion molecule holds great potential as a target for tumor therapy, yet the development of a therapeutic antibody specifically targeting L1 cell adhesion molecule is still required.

Antibody-drug conjugate belongs to a novel class of anti-cancer bio-missile drugs, which is composed of three components: an antibody, a cytotoxic, and a linker that connects the two. After the cytotoxin is conjugated to the monoclonal antibody by chemical conjugation, the antibody-drug conjugate utilizes the targeting ability of the monoclonal antibody to specifically recognize the receptor on the surface of cancer cell, and binds to the receptors. Once bound to the receptor, the antibody-drug conjugate is internalized into the cancer cell, and the cytotoxin is released by intracellular proteases to prevent the cancer cell from proliferation and kill the cancer cell. Antibody-drug conjugate technology integrates small molecule drugs and biologic proteins, which has the advantages of both, significantly enhances the efficacy and reduces the toxic and side effects, making it a next-generation therapeutic product.

The first clinically successful example of targeted antibody-drug conjugate is Gemtuzumab ozogamicin (Wyeth, trade name: Mylotarg). Mylotarg is the first monoclonal antibody-conjugated drug approved for market. The drug consists of anti-CD33 antibody, DNA-degrading drug Calicheamicin, and chemical linker AcBut. Mylotarg is a humanized anti-CD33 IgG4 antibody conjugated to the anti-tumor drug Calicheamicin for the treatment of acute myeloid leukemia. Mylotarg is the first generation of monoclonal antibody-coupled drugs, while there are three fatal defects in technology. First, the linker used to attach the cytotoxins was highly unstable, with a half-life of only 2 days, leading to undesired drug release and severe toxic effect in clinical application. Second, the antibody was conjugated to the linker via the amino group of lysine, while there are dozens of lysine on the surface of an antibody, this will result in random conjugation sites and therefore partially affects the efficacy. More importantly, the conjugation technology at that time was underdeveloped, only 50% of the antibodies has been successfully conjugated to the drug, resulting in less-than-ideal clinical efficacy. Third, the antibody used was an IgG4 antibody, which lacked antibody-dependent cell-mediated cytotoxicity (ADCC) and complement-dependent cytotoxicity (CDC). As a result, Mylotarg was withdrawn from the market ten years after its listing due to its severe toxic and side effects and limited therapeutic efficacy.

The second clinically successful example of targeted antibody-drug conjugates is a novel drug for the treatment of Hodgkin's lymphoma. Despite this drug has only undergone second phase of clinical trials, it was approved by the U.S. FDAin 2011 due to its surprising efficacy. This drug is a novel targeted antibody-drug conjugate (ADC) developed by Seattle Genetics, which is capable of being used as targeted therapy for treating patients of two types of lymphoma expressing the CD30 antigen. The antibody-drug conjugate brentuximab consists of an anti-CD30 monoclonal antibody, a microtubule inhibitor (Monomethyl auristatin E, MMAE), and a dipeptide chemical linker. This antibody-drug conjugate is characterized by low side effects and high efficiency in inhibiting lymphoma. In the Phase II single-arm clinical trial, 102 patients aged from 15 to 77 years old (median age of 31 years old) with relapsed or refractory Hodgkin's lymphoma were treated with brentuximab for a median of 9 cycles. The overall response rate was 73%, and the median course of treatment was 6.7 months. The complete response rate was 34%, and the median course of treatment was 20.5 months. 40% of the patients that received treatment achieved partial responses. The most common adverse effect was peripheral neuropathy. The success of this drug demonstrates the technical feasibility and promising future of targeted antibody-drug conjugate.

Another successful example of targeted antibody-drug conjugates is T-DM1 against malignant breast cancer, developed by Genentech Inc. The monoclonal antibody of this antibody-drug conjugate is against the HER2 (ErbB2) on the surface of breast cancer cells, and the conjugated cytotoxic agent is the microtubule inhibitor DM1. Results of Phase III clinical trials of this drug showed more effective than chemotherapy and less toxic and lower side effects. Patients with breast cancer have previously received treatment of herceptin and taxane chemo-therapeutic drug, but the disease is still progressing. But the treatment of antibody-drug conjugate can significantly prolong the survival time of HER2-positive breast cancer patients without worsening the disease. Based on its promising efficacy, the U.S. Food and Drug Administration (FDA) has approved T-DM1 on February 22, 2013 for the treatment of patients having HER2-positive advanced metastatic breast cancer. Especially in 2019, polatuzumab vedotin (trade name Polivy), enfortumab vedotin (trade name Padcev), and fam-trastuzumab deruxtecan (trade name Enhertu) have been approved for market, which has further invigorated the enthusiasm of pharmaceutical industries focusing on development of novel ADC therapies.

### Summary

The present disclosure provides a L1CAM-targeting antibody-drug conjugate, wherein the antibody-drug conjugate comprises an antibody or antigen-binding fragment conjugated to one or more therapeutic agents, wherein the antibody or antigen-binding fragment comprises a light chain variable region having light chain complementarity determining region LCDR1, LCDR2, and LCDR3, and/or a heavy chain variable region having heavy chain complementarity determining region HCDR1, HCDR2, and HCDR3, wherein the heavy chain variable region has a HCDR1, a HCDR2, and a HCDR3 having at least 80%, 90%, or 100% identity with the HCDR1, HCDR2, and HCDR3 of the heavy chain variable region sequence selected from the group consisting of SEQ ID NO:21, SEQ ID NO:23, SEQ ID NO:25, SEQ ID NO:27, SEQ ID NO:29, and SEQ ID NO:31; the light chain variable region has a LCDR1, a LCDR2, and a LCDR3 having at least 80% identity with the LCDR1, LCDR2 and LCDR3 of the light chain variable region sequence selected from the group consisting of SEQ ID NO:22, SEQ ID NO:24, SEQ ID NO:26, SEQ ID NO:28, SEQ ID NO:30, and SEQ ID NO:32, wherein the therapeutic agents include, but are not limited to, cytotoxic drugs, microtubule inhibitors, DNA-damaging agents, immunopotentiating agents, and radioactive isotopes, wherein the therapeutic agent is conjugated to the antibody or antigen-binding fragment via a linker.

In one particular embodiment, wherein the heavy chain variable region and the light chain variable region have a set of CDRs selected from the group consisting of:
(1) a HCDR1, a HCDR2, and a HCDR3 having at least 80%, at least 90%, or 100% identity to the HCDR1, HCDR2, and HCDR3 of the heavy chain variable sequence of SEQ ID NO:21, and a LCDR1, a LCDR2, and a LCDR3 having at least 80%, at least 90%, or 100% identity to the LCDR1, LCDR2, and LCDR3 of the light chain variable region sequence of SEQ ID NO:22;
(2) a HCDR1, a HCDR2, and a HCDR3 having at least 80%, at least 90%, or 100% identity to the HCDR1, HCDR2, and HCDR3 of the heavy chain variable sequence of SEQ ID NO:23, and a LCDR1, a LCDR2, and a LCDR3 having at least 80%, at least 90%, or 100% identity to the LCDR1, LCDR2, and LCDR3 of the light chain variable region sequence of SEQ ID NO:24;
(3) a HCDR1, a HCDR2, and a HCDR3 having at least 80%, at least 90%, or 100% identity to the HCDR1, HCDR2, and HCDR3 of the heavy chain variable sequence of SEQ ID NO:25, and a LCDR1, a LCDR2, and a LCDR3 having at least 80%, at least 90%, or 100% identity to the LCDR1, LCDR2, and LCDR3 of the light chain variable region sequence of SEQ ID NO:26;
(4) a HCDR1, a HCDR2, and a HCDR3 having at least 80%, at least 90%, or 100% identity to the HCDR1, HCDR2, and HCDR3 of the heavy chain variable sequence of SEQ ID NO:27, and a LCDR1, a LCDR2, and a LCDR3 having at least 80%, at least 90%, or 100% identity to the LCDR1, LCDR2, and LCDR3 of the light chain variable region sequence of SEQ ID NO:28;
(5) a HCDR1, a HCDR2, and a HCDR3 having at least 80%, at least 90%, or 100% identity to the HCDR1, HCDR2, and HCDR3 of the heavy chain variable sequence of SEQ ID NO:29, and a LCDR1, a LCDR2, and a LCDR3 having at least 80%, at least 90%, or 100% identity to the LCDR1, LCDR2, and LCDR3 of the light chain variable region sequence of SEQ ID NO:30;
(6) a HCDR1, a HCDR2, and a HCDR3 having at least 80%, at least 90%, or 100% identity to the HCDR1, HCDR2, and HCDR3 of the heavy chain variable sequence of SEQ ID NO:31, and a LCDR1, a LCDR2, and a LCDR3 having at least 80%, at least 90%, or 100% identity to the LCDR1, LCDR2, and LCDR3 of the light chain variable region sequence of SEQ ID NO:32.

In one particular embodiment, wherein:
(1) the HCDR1 has at least 80%, at least 90%, or 100% identity with the sequence selected from the group consisting of SEQ ID NO:1, SEQ ID NO:7, SEQ ID NO:10, SEQ ID NO:16;
(2) the HCDR2 has at least 80%, at least 90%, or 100% identity with the sequence selected from the group consisting of SEQ ID NO:2, SEQ ID NO:8, SEQ ID NO: 11, SEQ ID NO: 17;
(3) the HCDR3 has at least 80%, at least 90%, or 100% identity with the sequence selected from the group consisting of SEQ ID NO:3, SEQ ID NO:9, SEQ ID NO: 12, SEQ ID NO: 18;
(4) the LCDR1 has at least 80%, at least 90%, or 100% identity with the sequence selected from the group consisting of SEQ ID NO:4, SEQ ID NO: 13;
(5) the LCDR2 has at least 80%, at least 90%, or 100% identity with the sequence selected from the group consisting of SEQ ID NO:5, SEQ ID NO: 14, SEQ ID NO: 19;
(6) the LCDR3 has at least 80%, at least 90%, or 100% identity with the sequence selected from the group consisting of SEQ ID NO:6, SEQ ID NO: 15, SEQ ID NO:20.

In one particular embodiment, wherein:
(1) the HCDR1 comprises the amino acid sequence as set forth in SEQ ID NO: 1;
(2) the HCDR2 comprises the amino acid sequence as set forth in SEQ ID NO:2;
(3) the HCDR3 comprises the amino acid sequence as set forth in SEQ ID NO:3;
(4) the LCDR1 comprises the amino acid sequence as set forth in SEQ ID NO:4;
(5) the LCDR2 comprises the amino acid sequence as set forth in SEQ ID NO:5;
(6) the LCDR3 comprises the amino acid sequence as set forth in SEQ ID NO:6.

In one particular embodiment, wherein:
(1) the HCDR1 comprises the amino acid sequence as set forth in SEQ ID NO:7;
(2) the HCDR2 comprises the amino acid sequence as set forth in SEQ ID NO:8;
(3) the HCDR3 comprises the amino acid sequence as set forth in SEQ ID NO:9;
(4) the LCDR1 comprises the amino acid sequence as set forth in SEQ ID NO:4;
(5) the LCDR2 comprises the amino acid sequence as set forth in SEQ ID NO:5;
(6) the LCDR3 comprises the amino acid sequence as set forth in SEQ ID NO:6.

In one particular embodiment, wherein:
(1) the HCDR1 comprises the amino acid sequence as set forth in SEQ ID NO: 10;
(2) the HCDR2 comprises the amino acid sequence as set forth in SEQ ID NO: 11;
(3) the HCDR3 comprises the amino acid sequence as set forth in SEQ ID NO: 12;
(4) the LCDR1 comprises the amino acid sequence as set forth in SEQ ID NO: 13;
(5) the LCDR2 comprises the amino acid sequence as set forth in SEQ ID NO: 14;
(6) the LCDR3 comprises the amino acid sequence as set forth in SEQ ID NO: 15.

In one particular embodiment, wherein:
(1) the HCDR1 comprises the amino acid sequence as set forth in SEQ ID NO: 16;
(2) the HCDR2 comprises the amino acid sequence as set forth in SEQ ID NO: 17;
(3) the HCDR3 comprises the amino acid sequence as set forth in SEQ ID NO:18;
(4) the LCDR1 comprises the amino acid sequence as set forth in SEQ ID NO:13;
(5) the LCDR2 comprises the amino acid sequence as set forth in SEQ ID NO:19;
(6) the LCDR3 comprises the amino acid sequence as set forth in SEQ ID NO:20.

In one particular embodiment, wherein: the heavy chain variable region comprises an amino acid sequence of any one selected from the group consisting of:
(1) the amino acid sequence having at least 90%, at least 95%, or 100% sequence identity with SEQ ID NO:21;
(2) the amino acid sequence having at least 90%, at least 95%, or 100% sequence identity with SEQ ID NO:23;
(3) the amino acid sequence having at least 90%, at least 95%, or 100% sequence identity with SEQ ID NO:25;
(4) the amino acid sequence having at least 90%, at least 95%, or 100% sequence identity with SEQ ID NO:27;
(5) the amino acid sequence having at least 90%, at least 95%, or 100% sequence identity with SEQ ID NO:29;
(6) the amino acid sequence having at least 90%, at least 95%, or 100% sequence identity with SEQ ID NO:31.

In one particular embodiment, wherein the light chain variable region comprises an amino acid sequence of any one selected from the group consisting of:
(1) the amino acid sequence having at least 90%, at least 95%, or 100% sequence identity with SEQ ID NO:22;
(2) the amino acid sequence having at least 90%, at least 95%, or 100% sequence identity with SEQ ID NO:24;
(3) the amino acid sequence having at least 90%, at least 95%, or 100% sequence identity with SEQ ID NO:26;
(4) the amino acid sequence having at least 90%, at least 95%, or 100% sequence identity with SEQ ID NO:28;
(5) the amino acid sequence having at least 90%, at least 95%, or 100% sequence identity with SEQ ID NO:30;
(6) the amino acid sequence having at least 90%, at least 95%, or 100% sequence identity with SEQ ID NO:32.

In one particular embodiment, wherein the antibody is a monoclonal antibody, a bispecific antibody, a multispecific antibody, a recombinant antibody, a chimeric antibody, a bivalent antibody, an anti-idiotype antibody, or a fusion protein.

In one particular embodiment, wherein the linker is linked to the antibody via a thiol group.

In one particular embodiment, wherein the therapeutic agent includes, but not limited to, any one of auristatin cytotoxic molecules (e.g., MMAE and MMAF), maytansinoid cytotoxic molecules (e.g., DM1 and DM4), ansamycins derivatives pyrrolobenzodiazepines (PBDs), camptothecins and camptothecin derivatives (e.g., Exatecan and Dxd), or any combination thereof.

The present invention provides an antibody-drug conjugate having the following structure of formula (I): 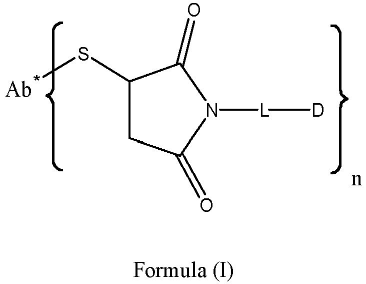
wherein "Ab" refers to an antibody or antigen-binding fragment selected from any of the antibodies or antigen-binding fragments as described herein;
wherein, L refers to a cleavable or non-cleavable linker;
wherein, D refers to a therapeutic agent;
wherein, n refers to the drug-to-antibody ratio (DAR), preferably ranging from 1 to 8.

In a preferred embodiment, wherein L refers to a cleavable linker.

In a preferred embodiment, wherein L refers to -L₁-L₂-L₃-, wherein L₁ refers to any one of: -(CH₂)ₙ-X-Y-(CH₂)ₘ-, -X (CH₂)ₙ-O-(CH₂CH₂O)ₚ-(CH₂)ₘ-Y-, -(CH₂)ₙ-X-, -X- (CH₂)ₘ-Y-, or combination thereof, wherein X and Y each independently refers to -C(O)-, O, -CR¹R², -NR¹-, S, or is not present, wherein R¹ and R² each independently refers to hydrogen, C₁-C₆ alkyl, or C₃-C₆ cycloalkyl, wherein m, n, and p each independently refers to an integer from 0 to 10;
wherein L₂ refers to an amino acid residue;
wherein L₃ is not present or refers to -C(O)-, or -PAB-C(O);
wherein PAB refers to p-aminobenzyloxy.

In a preferred embodiment, wherein L₂ referes to any one of -Val-Cit-, -Val-Ala-, -Gly-Gly-Phe-Gly-, Ala-Ala-Asn-, -Val-Lys, -Phe-Lys, -Phe-Cit, -Phe-Arg-, -Phe-Ala-, -Ala-Lys, -Leu-Cit-, -Ile-Cit-, -Trp-Cit-, -D-Phe-LPhe-Lys-, -Phe-Phe-Lys-, -D-Phe-Phe-Lys-, -Gly-Phe-Lys-, -Gly-Phe-Leu-Gly-, Ala-Leu-Ala-Leu or any combination thereof.

In a preferred embodiment, wherein L refers to -(CH₂)₅-Val-Cit-PAB-C(O)-, -(CH₂)₅-Val-Ala-PAB-C(O)-.

In a preferred embodiment, wherein the therapeutic agents selected from any one of MMAE, MMAF, Dxd, DM1, DM4 or any combination thereof.

In addition, the present invention provides an antibody-drug conjugate having the following structure: 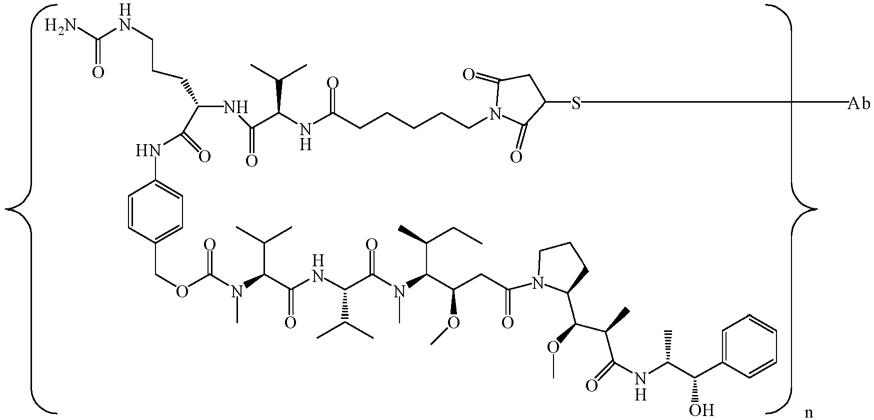 or 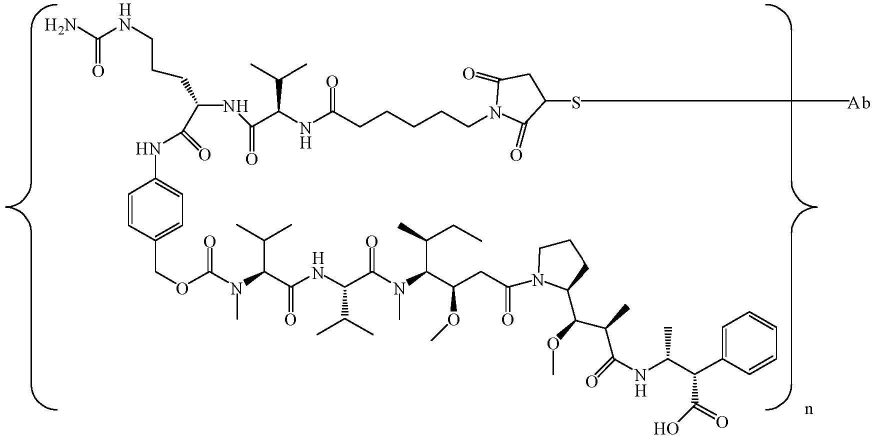 wherein "Ab" refers to an antibody or antigen-binding fragment selected from any of the antibodies or antigen-binding fragments as described above of the invention, wherein n refers to drug-to-antibody ratio (DAR), preferably ranging from 1 to 8.

In addition, the present invention also provides a pharmaceutical composition comprising an antibody-drug conjugate as disclosed herein and a pharmaceutically acceptable carrier.

### Description of Figures

Figure 1 The affinity to L1CAM on the surface of cells of the humanized anti-L1CAM antibody-drug conjugate (anti-L1CAM ADC).
Figure 2 The binding specificity to L1CAM on the surface of cells of humanized anti-L1CAM antibody-drug conjugate (anti-L1CAM ADC).
Figure 3 The cytotoxicity of the humanized anti-L1CAM antibody-drug conjugate (anti-L1CAM ADC) against HeLa cells.
Figure 4 The cytotoxicity of the humanized anti-L1CAM antibody-drug conjugate (anti-L1CAM ADC) against HeLa L1CAM KO cells.

### Detailed Description

In order to better understand the disclosure, the definitions and explanations of the relevant terms are provided as follows. These definitions are intended to encompass grammatical equivalents and other variations.

The term "antibody" as used herein refers to a protein consisting of one or more polypeptides encoded by substantially all or part of a recognized immunoglobulin gene. Such immunoglobulin gene, for example, in human, comprising kappa (κ), lambda (λ), and a heavy chain locus, which comprise a plurality of genes encoding variable region, as well as genes encoding constant region mu (µ), delta (δ), gamma (γ), epsilon (ε), and alpha (α) of IgM, IgD, IgG, IgE, and IgA isotypes, respectively. The antibody disclosed herein is intended to include full-length antibody and antibody fragment, as well as naturally occurring antibody from any organism, engineered antibody, or those recombinantly produced for the purpose of experiment, therapy, or other purposes as further defined below. The term "antibody" encompasses antibody fragments, which are well known in the art, such as Fab, Fab', F(ab')2, Fv, scFv, or other sub-sequence of antibody for antigen-binding, or antibody fragments produced by modification of full-length antibodies or by those de novo synthesized antibodies using recombinant DNA technology. The term "antibody" includes monoclonal and polyclonal antibodies. The antibodies may be antagonists, agonists, neutralizing antibodies, inhibitory antibodies, or stimulatory antibodies. The antibodies of the present invention may be non-human antibodies, chimeric antibodies, humanized antibodies, or fully human antibodies.

The term "monoclonal antibody" as used herein refers to an antibody obtained from a substantially homogeneous population of antibodies, i.e., the population comprising unique antibodies are identical except for the presence of little potential mutations (e.g., natural mutations). Therefore, the term "monoclonal" indicates the nature of the antibody, i.e., it is not a mixture of irrelevant (discrete) antibodies. In contrast to polyclonal antibody preparations, which typically include antibodies against distinct antigenic determinants (epitopes), each monoclonal antibody of the monoclonal antibody preparations is against a single determinant on the antigen. In addition to the specificity, the advantage of monoclonal antibody preparations lies in the fact that they can typically avoid contamination by other immunoglobulins. The term "monoclonal" should not be construed as the antibody is required to be produced by any specific method. The term monoclonal antibody specifically includes humanized antibody.

The term "complementarity determining region" (CDR, e.g., CDR1, CDR2, and CDR3) when used in the disclosure refers to the amino acid residues in the variable region of an antibody, and the presence of these CDRs is essential for antigen binding. The "antigen-binding region" of the antibody typically exists within one or more hypervariable region(s) of the antibody, such as the CDR regions of CDR1, CDR2, and CDR3. Each complementarity determining region may comprise amino acid residues from the "complementarity determining region" as defined by Kabat, for example, residues 24-34 (L1), 50-56 (L2), and 89-97 (L3) in the light chain variable region, and residues 26-33 (H1), 51-57 (H2), and 96-105 (H3) in the heavy chain variable region (Kabat et al., Sequences of Proteins of Immunological Interest, 5th Ed., Public Health Service, National Institutes of Health, 1991). In some embodiments, the CDR regions as defined herein utilizing the Kabat scheme. The CDRs in the sequence of an antibody can also be identified according to other general definitions developed in the art (e.g., Chothia, IMGT, Contact numbering system, etc.) or by comparing the sequence with databases of known variable regions. Methods for identifying these regions are described in Kontermann and Dubel edited, Antibody Engineering, Springer, New York, NY, 2001; and Dinarello et al., Current Protocols in Immunology, John Wiley and Sons Inc., Hoboken, NJ, 2000. The same antibody as disclosed herein may have a different set of CDRs when determined by a different numbering scheme. The disclosure of the CDRs determined by one numbering scheme herein includes the disclosure of the CDRs that might be determined by other numbering scheme. It is considered that two antibodies having the same CDRs if their CDRs are identical when determined by the same scheme.

The term "antigen" as used herein is intended to refer to a compound, composition, or substance including the composition injected or absorbed into the animal, which is capable of stimulating production of antibodies or T-cell responses within an animal, and the antigens can be proteins, carbohydrates, lipids, or other pathogens.

The "functional fragment" or "antigen-binding fragment" of an antibody/immunoglobulin, as defined herein, is a fragment of an antibody/immunoglobulin that retains the antigen-binding region (e.g., the variable region of IgG). The "antigen-binding region" of an antibody typically exists in one or more hypervariable regions of the antibody, such as CDR1, CDR2, and/or CDR3 region; however, the variable "framework" region can also play an important role in antigen binding, for example, by providing a scaffold for the CDRs. Preferably, the "antigen-binding region" includes at least the amino acid residues 4-103 of variable light chain (VL) and the amino acid residues 5-109 of variable heavy chain (VH), more preferably, the amino acid residues 3-107 of VL and the amino acid residues 4-111 of VH, and even more preferably, the full-length VL and VH chains (the amino acids 1-109 of VL and the amino acids 1-113 of VH, numbered according to WO97/08320). The preferred immunoglobulin class used in the present invention is IgG.

The term "amino acid" as used herein refers to one of the 20 naturally occurring amino acids or any one of non-natural analogs, which can be located at a specifically defined position. The term "protein" as used herein refers to at least two covalently linked amino acids, which including proteins, polypeptides, oligopeptides, and peptides. A protein may be composed of naturally occurring amino acids and peptide bonds, or composed of synthetic peptide mimic structure, the peptide mimic is referred to as "analogs". Therefore, the "amino acid" or "peptide residue" as used herein refers to both naturally occurring and synthetic amino acids. For instance, for the purpose of the invention, homophenylalanine, citrulline, and norleucine are considered as the amino acids used for the purpose of the invention. "Amino acid" also includes imino acid residues such as proline and hydroxyproline. The side chains may be in (R) or (S) configuration. In preferred embodiments, amino acids are present in (S) or L-configuration. If a non-natural side chain is used, the non-amino acid substitution can be employed, such as to prevent or delay in vivo degradation.

The term "identity" as used herein is intended to refer to the similarity between two or more nucleotide sequences or amino acid sequences or referred to as sequence identity. Sequence identity is typically measured by the percentage of identity (or similarity or homology); the higher the percentage, the more similar the two sequences are. When sequences are aligned by using standard methods, homologs or variants will have a relatively high degree of sequence identity. Methods of sequence alignment for comparison are well established in the art. Various programs and alignment algorithms are described in: Smith and Waterman, Adv Appl. Math., 2: 482, 1981; Needleman and Wunsch, J. Mol. Biol. 48: 443, 1970; Pearson and Lipman, Proc. Natl. Acad. Sci. U.S.A. 85: 2444, 1988; Higgins and Sharp, Gene 73: 237-244, 1988; Higgins and Sharp, CABIOS 5: 151-153, 1989; Corpet et al., Nucleic Acids Research 16: 10881-10890, 1988; and Altschul et al., Nature Genet., 1994, 6: 119-129.

The NCBI Basic Local Alignment Search Tool (BLAST^{™}) (Altschul et al., J. Mol. Biol., 215: 403-410, 1990) is available from multiple sources, including National Center for Biotechnology Information (NCBI, Bethesda, MD) and on the Internet related to the programs for sequence analysis blastp, blastn, blastx, tblastn, and tblastx.

The term "nucleic acid" as used herein refers to a polymer consisting of nucleotide units (ribonucleotides, deoxyribonucleotide, relevant naturally occurring structural variants, and synthetic non-naturally occurring analogs thereof) linked by phosphodiester bonds. Therefore, the term includes nucleotide polymers, wherein the nucleotides and the bonds between them include non-naturally occurring synthetic analogs, for example but not limited to, phosphorothioate, phosphoramidate, methylphosphonate, chiral methylphosphonate, 2'-O-methyl ribonucleotides, peptide nucleic acids (PNA), and the like. These polynucleotides can be for example synthesized by using automated DNA synthesizer. The term "oligonucleotide" typically refers to a short polynucleotide, which is generally no longer than about 50 nucleotides. It should be understood that when a nucleotide sequence is represented by a DNA sequence (i.e., A, T, G, C), it also includes the case of RNA sequences in which "U" replaces "T" (i.e., A, U, G, C).

The nucleotide sequences are described herein using conventional symbols: the 5'-end of a single-stranded nucleotide sequence is at the left-hand end, and the 5'-direction is referred to as the left-hand direction of a double-stranded nucleotide sequence. The direction in which nucleotides are added to the growing RNA transcript is referred to as the transcription direction (from 5' to 3'). The DNA strand that has the same sequence as the mRNA is referred to as the coding strand.

The term "encode", "encoding" or "encoded" as used herein refers to the inherent property of a specific nucleotide sequence within a polynucleotide, such as a gene, cDNA, or mRNA, which serves as a template for the synthesis of other polymers and macromolecules in biological processes with definite nucleotide sequences, or refers to defined amino acid sequences and the resulting biological properties. Therefore, if transcription and translation of the mRNA generated by a gene result in the production of a protein within a cell or other biological system, the gene encodes the protein. The coding strand (which has the same nucleotide sequence as the mRNA and is usually provided in the sequence listing) and the non-coding strand (which serves as the transcription template, such as gene or cDNA) can be referred to as encoding the protein or other products of the gene or cDNA. Unless otherwise indicated, the "nucleotide sequence encoding an amino acid sequence" includes all nucleotide sequences that are degenerate forms of each other and encode the same amino acid sequence. The nucleotide sequences encoding protein and RNA may comprise introns.

The term "plasmid" as used herein refers to a plasmid that has been artificially constructed on the basis of native plasmids to adapt to laboratory procedures. Nucleic acid molecules can be introduced into a host cell, thereby producing a transformed host cell. The plasmid may comprise nucleic acid sequences that allow for its replication within the host cell, such as an origin of replication, and may also comprise one or more selective marker gene(s), as well as other genetic elements known in the art.

The term "host cell" as used herein, also known as recipient cell, refers to a cell that receives an exogenous gene through transformation and transduction (infection).

The term "pharmaceutically acceptable carrier" as used herein means a conventional pharmaceutically acceptable carrier known in the art. The compositions and formulations that suitable for the drug delivery of one or more therapeutic compounds or molecules (e.g., one or more antibodies), as well as additional pharmaceutical agents are described in Remington's Pharmaceutical Sciences, E.W.Martin, Mack Publishing Co., Easton, Pa., 15th Edition (1975).

As used herein, the "diagnosis" of a disease refers to the determination of the condition and state of its progression for a patient after examination has been conducted. The term "prevent", "prevention" or "preventing" with reference to a certain disease, refers to the inhibition of the complete development of the disease. "Treatment", "treating" or "treated" refers to the therapeutic intervention aimed at improving the signs or symptoms of the disease or pathological condition after it has begun to develop.

The term "administering", "administer" or "administered" as used herein means the process of introducing the substance into a subject through an appropriate route. For example, if the selected route is intravenous, the composition will be administered by introducing the substance into the subject's vein.

The term "prophylactically/therapeutically effective amount/dose" as used herein means a certain amount of a given agent that is effective to achieve some desired effects in the subject being treated with the agent. The precise dose will depend on the purpose of treatment and can be determined by those skilled in the art using well-established techniques. The dose range may be from 0.01 to 100 mg/kg body weight or greater, such as 0.1, 1, 10, or 50 mg/kg body weight, with a preferred range of 1-10 mg/kg. As is known in the art, adjustments may be necessary with regard to antibody or Fc fusion degradation, systemic or local drug delivery, and nascent protein enzymatic synthesis rates, as well as age, body weight, general state of health, gender, diet, the timing of administration, drug interaction, and the severity of the condition, and can be determined by those skilled in the art through conventional experimental methods. Such agents include the monomeric Fc domain molecules described herein. In one non-limiting example, this can be an amount of HIV-specific monomeric Fc domain (or HIV-specific CH3 domain molecule) for used in the prevention, treatment, or improvement of HIV infection. Optimally, a therapeutically effective amount of antibody is the amount sufficient to prevent, treat, or improve the infection or disease, such as that caused by HIV infection in the subject, without causing significant cytotoxic effects in the subject. The therapeutically effective amount of the agent for used in the prevention, improvement, and/or treatment of a subject will depend on the subject being treated, the type of pain and severity, and the approach of administering the therapeutic composition.

The term "cancer" as used herein refers to a solid tumor or hematogenous cancer. The solid tumor described herein is a sarcoma or carcinoma, for example, a fibrosarcoma, mucinous sarcoma, liposarcoma, chondrosarcoma, osteosarcoma or another sarcoma, synovial sarcoma, mesothelioma, Ewing's sarcoma, leiomyosarcoma, rhabdomyosarcoma, colon cancer, lymphoid malignancy, pancreatic cancer, breast cancer, lung cancer, ovarian cancer, prostate cancer, hepatocellular carcinoma, squamous cell carcinoma, basal cell carcinoma, adenocarcinoma, hidradenocarcinoma, sebaceous carcinoma, papillary carcinoma, papillary adenocarcinoma, medullary carcinoma, bronchogenic carcinoma, renal cell carcinoma, hepatocellular carcinoma, cholangiocarcinoma, choriocarcinoma, nephroblastoma, cervical cancer, testicular tumor, bladder cancer, or tumor of central nervous system (such as glioma, astrocytoma, medulloblastoma, craniopharyngioma, ependymoma, pinealoma, hemangioblastoma, acoustic neuroma, oligodendroglioma, hemangioma, melanoma, neuroblastoma, or retinoblastoma). The hematogenous cancer described herein is a leukemia, such as acute leukemia (e.g., acute lymphocytic leukemia, acute myeloid leukemia, acute myelogenous leukemia, and myeloblastic, promyelocytic, myelomonocytic, monocytic, and erythroid leukemia); chronic leukemia (e.g., chronic granulocytic (granulocyte) leukemia, chronic granulocytic leukemia, chronic lymphocytic leukemia), polycythemia vera, lymphoma, Hodgkin's disease, non-Hodgkin's lymphoma (indolent and aggressive), multiple myeloma, Waldenström's macroglobulinemia, heavy chain disease, myelodysplastic syndrome, hairy cell leukemia, or myelodysplasia.

Unless otherwise defined, technical and scientific terms used in connection with the present disclosure shall have the meanings that commonly understood by those of ordinary skill in the art. Unless otherwise clearly dictated by the context, as used in this specification and the appended claims, singular terms "a", "an" and "the" include plural referents. It should also be understood that all base sizes or amino acid sizes for nucleic acids or polypeptides, as well as all molecular weights or molecular weight values, are approximation and are provided for descriptive purposes. Although methods and materials similar or equivalent to those described herein may be used in practicing or testing the present disclosure, suitable methods and materials are described below. The term "comprising" means "including". All publications, patent applications, patents, and other references cited herein are incorporated by reference in their entirety. In the event of any conflict, the present specification (including the definitions of terms) shall take precedence. Additionally, the materials, methods, and examples provided herein are for illustrative purposes only and are not intended to be limiting.

### Anti-L1CAM antibody

The antibodies of the invention include antibodies of all classes (i.e., IgA, IgD, IgE, IgG, and IgM) and all subclasses (i.e., IgG1, IgG2, IgG3, IgG4, IgA1, and IgA2). The antibodies may be chimeric or humanized monoclonal antibodies.

The antibody typically comprises a heavy chain variable region (VH) and a light chain variable region (VL), and the VH and VL regions may be further subdivided into hypervariable regions (referred to as complementarity-determining regions (CDRs)) which are separated by relatively conserved regions (referred to as framework regions (FRs)). Each VH and VL consists of 3 CDRs and 4 FRs arranged sequentially in the order of: from N-terminal to C-terminal, FR1, CDR1, FR2, CDR2, FR3, CDR3, and FR4. The variable regions (VH and VL) of each heavy chain/light chain pair form the antigen-binding site respectively.

In some embodiments, the antibody or antigen-binding portion thereof comprises: a heavy chain CDR1 comprising the amino acid sequence of SEQ ID NO: 1, a heavy chain CDR2 comprising the amino acid sequence of SEQ ID NO: 2, a heavy chain CDR3 comprising the amino acid sequence of SEQ ID NO: 3, a light chain CDR1 comprising the amino acid sequence of SEQ ID NO: 4, a light chain CDR2 comprising the amino acid sequence of SEQ ID NO: 5, and a light chain CDR3 comprising the amino acid sequence of SEQ ID NO: 6 (as shown in the antibody hz26A12C1H4L3).

In some embodiments, the antibody or antigen-binding portion thereof comprises: a heavy chain CDR1 comprising the amino acid sequence of SEQ ID NO: 7, a heavy chain CDR2 comprising the amino acid sequence of SEQ ID NO: 8, a heavy chain CDR3 comprising the amino acid sequence of SEQ ID NO: 9, a light chain CDR1 comprising the amino acid sequence of SEQ ID NO: 4, a light chain CDR2 comprising the amino acid sequence of SEQ ID NO: 5, and a light chain CDR3 comprising the amino acid sequence of SEQ ID NO: 6 (as shown in the antibody hz46F1D5H4L3).

In some embodiments, the antibody or antigen-binding portion thereof comprises: a heavy chain CDR1 comprising the amino acid sequence of SEQ ID NO:10, a heavy chain CDR2 comprising the amino acid sequence of SEQ ID NO: 11, a heavy chain CDR3 comprising the amino acid sequence of SEQ ID NO: 12, a light chain CDR1 comprising the amino acid sequence of SEQ ID NO: 13, a light chain CDR2 comprising the amino acid sequence of SEQ ID NO: 14, and a light chain CDR3 comprising the amino acid sequence of SEQ ID NO: 15 (as shown in the antibody hz49E10H1H5L6).

In some embodiments, the antibody or antigen-binding portion thereof comprises: a heavy chain CDR1 comprising the amino acid sequence of SEQ ID NO:16, a heavy chain CDR2 comprising the amino acid sequence of SEQ ID NO: 17, a heavy chain CDR3 comprising the amino acid sequence of SEQ ID NO: 18, a light chain CDR1 comprising the amino acid sequence of SEQ ID NO: 13, a light chain CDR2 comprising the amino acid sequence of SEQ ID NO: 14, and a light chain CDR3 comprising the amino acid sequence of SEQ ID NO: 15 (as shown in the antibody hz49E10H1H5L7).

In some embodiments, the antibody or antigen-binding portion thereof comprises: a heavy chain CDR1 comprising the amino acid sequence of SEQ ID NO:16, a heavy chain CDR2 comprising the amino acid sequence of SEQ ID NO: 17, a heavy chain CDR3 comprising the amino acid sequence of SEQ ID NO: 18, a light chain CDR1 comprising the amino acid sequence of SEQ ID NO: 13, a light chain CDR2 comprising the amino acid sequence of SEQ ID NO: 19, and a light chain CDR3 comprising the amino acid sequence of SEQ ID NO: 20 (as shown in the antibody hz103E9B3H5L5 or hz103E9B3H6L5).

In some embodiments, the antibody or antigen-binding portion thereof comprises a heavy chain variable region (VH) and a light chain variable region (VL):
the VH comprises: (i) an amino acid sequence as set forth in any one of SEQ ID NO: 21, 23, 25, 27, 29, or 31; or (ii) an amino acid sequence that is at least 85%, 90%, or 95% identical to any one of the amino acid sequences set forth in SEQ ID NO: 21, 23, 25, 27, 29, or 31 and retains specific binding affinity to L1CAM when associated with the VL region; and
the VL comprises: (i) an amino acid sequence as set forth in any one of SEQ ID NO: 22, 24, 26, 28, 30, or 32; or (ii) an amino acid sequence that is at least 85%, 90%, or 95% identical to any one of the amino acid sequences set forth in SEQ ID NO: 22, 24, 26, 28, 30, or 32 and retains specific binding affinity to L1CAM when associated with the VH region.

Preferably, the variant whose amino acid sequence is at least 85%, 90%, or 95% identical as described above, includes mutations occurring in the framework regions other than in the CDR regions, for example, there may be one or more (e.g., 1, 2, 3, 4, 5, 6, 7, 8, 9, or 10) amino acid additions, deletions, and/or substitutions in the framework regions. Preferably, the substitution is conservative substitution.

The term "conservative substitution" as used herein refers to amino acid substitutions which would not disadvantageously affect or change the essential properties of a protein/polypeptide comprising the amino acid sequence. For example, a conservative substitution may be introduced by standard techniques known in the art, such as site-directed mutagenesis and PCR-mediated mutagenesis. Conservative amino acid substitutions include substitutions wherein an amino acid residue is substituted with another amino acid residue having a similar side chain, for example, a residue physically or functionally similar (such as, having similar size, shape, charge, chemical property including the capability of forming covalent bond or hydrogen bonds, etc.) to the corresponding amino acid residue. The families of amino acid residues having similar side chains have been defined in the art. These families include amino acids having alkaline side chains (for example, lysine, arginine and histidine), amino acids having acidic side chains (for example, aspartic acid and glutamic acid), amino acids having uncharged polar side chains (for example, glycine, asparagine, glutamine, serine, threonine, tyrosine, cysteine, tryptophan), amino acids having nonpolar side chains (for example, alanine, valine, leucine, isoleucine, proline, phenylalanine, methionine), amino acids having β-branched side chains (such as threonine, valine, isoleucine), and amino acids having aromatic side chains (for example, tyrosine, phenylalanine, tryptophan, histidine). Therefore, a corresponding amino acid residue is preferably substituted with another amino acid residue from the same side-chain family. Methods for identifying amino acid conservative substitutions are well known in the art (see, for example, Brummell et al., Biochem. 32: 1180-1187 (1993); Kobayashi et al., Protein Eng. 12(10): 879-884 (1999); and Burks et al., Proc. Natl. Acad. Sci. USA 94: 412-417 (1997), which are incorporated herein by reference).

The antibodies and antigen-binding fragments provided herein may further comprise a human IgG constant region, the human IgG constant region comprises an Fc region and optionally a hinge region. The human IgG constant region may be a human IgG1, IgG2, IgG3, or IgG4 constant region. In some embodiments, the antibody comprises a human IgG1 or IgG4 Fc region. The Fc region may be a wild-type Fc region, or the Fc region may comprise one or more amino acid modifications that change the antibody-dependent cell-mediated cytotoxicity (ADCC) or other effector function (such as Leu234Ala/Leu235Ala or LALA substitution).

### Linker-drug moiety

The use of antibody-drug conjugates for local delivery of cytotoxic or cytostatic agents, i.e. drugs to kill or inhibit tumor cells in the treatment of cancer allows targeted delivery of the drug moiety to tumors, and intracellular accumulation therein, where systemic administration of unconjugated drug agents may result in unacceptable levels of toxicity to normal cells as well as the tumor cells sought to be eliminated (Thorpe, (1985) "Antibody Carriers Of Cytotoxic Agents In Cancer Therapy: A Review", in Monoclonal Antibodies '84: Biological And Clinical Applications, A. Pinchera et al. (eds), pp. 475-506). Both polyclonal antibodies and monoclonal antibodies have been reported as useful in these strategies (Rowland et al., (1986) Cancer Immunol. Immunother., 21:183-87).

Drugs that can be used in the ADCs disclosed herein include chemotherapeutic agents such as daunomycin, adriamycin, methotrexate, and vindesine; toxins, for example bacterial toxins such as diphtheria toxin, plant toxins such as ricin, small molecule toxins such as geldanamycin, maytansinoids, and calicheamicin; auristatin peptides, auristatin E (AE), and monomethyl auristatin E (MMAE), which are synthetic analogs of dolastatin. MMAE is a synthetic derivative of dolastatin 10, a natural cytostatic pseudo peptide. The toxins may effect their cytotoxic and cytostatic effects by mechanisms including tubulin binding, DNA binding, or topoisomerase inhibition. When conjugated to large antibodies or protein receptor ligands, some cytotoxic drugs tend to be inactive or less active.

There are no specific limitations to the drug and linker which can be used in the conjugation process of the present disclosure, as long as the drug molecule has an antitumor, antiviral or antimicrobial effect and contains at least one substituted group or a partial structure allowing connection to a linker structure, and the linker contains at least two reactive groups, one of which can covalently bond a drug molecule and the other of which can covalently couple to an antibody. Preferably, the linker is susceptible to -SH attack from the antibody and capable of forming a linkage with the antibody.

Depending on the desired drug and the selected linker, those skilled in the art can select a suitable method for coupling them together. For example, some conventional coupling methods, such as amine coupling methods, may be used to form the desired drug-linker complex which still contains reactive groups for conjugating to the antibodies through covalent linkage. A drug-maleimide complex (i.e., maleimide linking drug) is taken as an example of the payload bearing reactive group in the present disclosure.

In an embodiment, the drug may include, but not limited to, cytotoxic reagents, such as chemotherapeutic agents, immunotherapeutic agents and the like, antiviral agents or antimicrobial agents. In an embodiment, the drug to be conjugated with an antibody may be selected from, but not limited to, MMAE (monomethyl auristatin E), MMAD (monomethyl auristatin D), MMAF (monomethyl auristatin F), and the like.

Most common reactive group capable of bonding to thiol group in ADC preparation is maleimide. Additionally, organic bromides, iodides also are frequently used.

Enzymatically active toxins and fragments thereof which can be used include diphtheria A chain, nonbinding active fragments of diphtheria toxin, exotoxin A chain (from *Pseudomonas aeruginosa*)*,* ricin A chain, abrin A chain, modeccin A chain, alpha-sarcin, Aleurites fordii proteins,, dianthin proteins, Phytolacca americana proteins (PAPI, PAPII, and PAP-S), momordica charantia inhibitors, curcin, crotin, saponaria officinalis inhibitors, gelonin, mitogellin, restrictocin, phenomycin, enomycin, and the trichothecenes. See, for example, WO 93/21232 published October 28, 1993. A variety of radioactive isotopes are available for the production of radioconjugated antibodies. Examples include 212Bi, 131I, 131In, 90Y, and 186Re. One or more small molecule toxins, such as a calicheamicin, maytansinoids, dolastatins, auristatins, a trichothecene, and CC1065, and the derivatives of these toxins that have toxin activity, can be conjugated to an antibody by the methods disclosed herein.

Maytansine compounds suitable for use as maytansinoid drug moieties are well known in the art and can be isolated from natural sources according to known methods, produced using genetic engineering techniques (see Yu et al. (2002) PNAS 99:7968-7973), or maytansinol and maytansinol analogues prepared synthetically according to known methods. Suitable maytansinoids are disclosed, for example, in U.S. Patent No. 5,208,020. Preferred maytansinoids include maytansinol and maytansinol analogues modified in the aromatic ring or at other positions of the maytansinol molecule, such as various maytansinol esters.

Dolastatins and auristatins have been shown to interfere with microtubule dynamics, GTP hydrolysis, and nuclear and cellular division and have anticancer and antifungal activity (Pettit et al., Antimicrob. Agents Chemother. 42:2961-2965, 1998). The dolastatin or auristatin drug moiety may be attached to the antibody through the N (amino) terminus or the C (carboxyl) terminus of the peptidic drug moiety (WO 02/088172). Exemplary embodiments comprising MMAE or MMAF and various linker components are shown below. For example, VcMMAE (Mc-vc-PABC-MMAE) is obtained by using MMAE linked via p-aminobenzyloxycarbonyl ("PABC") to the lysosomally cleavable dipeptide valine-citrulline (vc) and a thiolreactive maleimidocaproyl spacer (Mc).

Conjugates of the antibody and cytotoxic agent may be made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al (1987) Science, 238:1098. Carbon-14-labeled 1-isothiocyanatobenzyl-3-methyldiethylene triaminepentaacetic acid (MX-DTPA) is an exemplary chelating agent for conjugation of radionucleotide to the antibody (WO94/11026).

The present disclosure also encompasses exemplary embodiments comprising MMAE or MMAF and various linker components. For example, VcMMAE (Mc-vc-PABC-MMAE) is obtained by using MMAE linked via p-aminobenzyloxycarbonyl ("PABC") to the lysosomally cleavable dipeptide valine-citrulline (vc) and a thiolreactive maleimidocaproyl spacer (MC).

### Applications of the ADCs

In one aspect, the present disclosure relates to a use of the antibody-drug conjugates prepared by the methods mentioned above in the manufacture of a pharmaceutical composition or a kit for the treatment of a condition or disease in a subject. In one aspect, the present disclosure also relates to a method for treating a subject with a cancer, comprising administering to the subject in need thereof a therapeutically effective amount of the antibody-drug conjugates to treat or prevent the condition or disease. The subject may be a mammal, such as a human. The condition or disease to be treated may be a cancer, particularly the cancer associated with L1CAM. In certain embodiments, the cancer may be selected from breast cancer, cholangiocarcinoma, melanoma, pancreatic cancer, glioma, lymphoma, lung cancer, renal cancer, prostate cancer, fibrosarcoma, colon adenocarcinoma, liver cancer, or ovarian cancer.

### Example

The present invention will be further described by way of embodiment, but does not limit the present invention to the scope of the described embodiments.

### Example 1: Production of monoclonal antibodies against L1CAM

To generate L1CAM antibodies, Balb/c mice were immunized with recombinantly expressed human L1CAM antigen fragments. During the immune operation, blood was collected from the mice regularly to obtain serum samples and monitor the immune response. Mice with sufficient titers of anti-L1CAM antibodies were used for fusion experiments. Splenocytes and/or lymph node cells from immunized mice were isolated and fused with mouse myeloma cell lines. By performing ELISA analysis with the recombinant protein of the extracellular domain of human L1CAM, hybridomas that can produce L1CAM-specific antibodies were screened out using HeLa cells that highly express human L1CAM and HEK293 cells that highly express monkey L1CAM, and were subcloned to obtain stable of hybridoma clones. After subcloning, the monoclonal hybridoma cells are subjected to antibody sequencing for further analytical testing.

### Example 2: Production of chimeric antibodies against L1CAM

Based on the results of monoclonal hybridoma cell antibody sequencing, human-mouse chimeric antibodies with CH1, CH2 and CH3 of human IgG1 as constant regions and VH and VL of murine anti-L1CAM monoclonal antibodies as variable regions were designed and recombinant expressed in mammalian cells and purified for further analysis and testing.

### Example 3: Production of a humanized antibodies against L1CAM

Mouse anti-L1CAM monoclonal antibodies are selected for humanization. The antibody sequences are aligned with human germline sequences to determine the best fitting model. Based on homology with the original mouse antibody sequences, the most matching human germline sequences are selected as templates for humanization. The CDRs from the mouse antibody sequences are grafted onto the template along with residues that preserve the upper and central core structures of the antibody. Optimized mutations are introduced into the framework region to produce variants of the humanized heavy chain variable region and the humanized light chain variable region, which are mixed and matched to provide multiple humanized antibody clones. They are recombinantly expressed in mammalian cells and purified for further analytical testing.

### Example 4: Determination of humanized anti-L1CAM antibody binding on the surface of HeLa cells

The experimental principle is as follows: HeLa cells express high levels of L1CAM protein on their surface. After incubating humanized L1CAM antibodies with the cells, the binding ability of the antibodies to L1CAM on the surface of HeLa cells can be determined by flow cytometry.

The experimental method is described as following:
Humanized anti-L1CAM antibodies (Human IgG1-Kappa) are diluted in complete medium, in a four-fold serial dilution, with 7 concentration gradient points.

HeLa cells are cultured in DMEM complete medium (Corning, 10-013-CVR) containing 10% FBS (Gibco, 10099-141) and 100U/mL penicillin-streptomycin mixture (Thermofisher, 15140122). When the cell coverage in the culture container reaches 80-90%, TrypLE (Thermofisher, 12604-013) is used to digest the HeLa cells from the culture container, and they are seeded in a 96-well plate (AXYGEN, P-96-450V-C) at a density of 2.2 × 10⁶ cells/ml, with 100,000 cells (45µL complete medium) per well. Then, 5 µL of the antibody dilution is added to each well, mixed gently, and the 96-well plate is incubated at 4°C for 1 hour. Then, 150 µL of PBS (Corning, 21-040-CVC) containing 2% FBS is added to each well, centrifuged at 1400 rpm for 4 minutes, the supernatant is removed, and washed once again. Then, 50 µL of Alexa Fluor 647-labeled goat anti-human fluorescent secondary antibody (Jacksonimmuno, 109-605-088) diluted at 1:800 is added to each well, and the 96-well plate is incubated at 4°C for 0.5 hours. The cells are washed twice with PBS solution containing 2% FBS, then resuspended in 120 µL of PBS, and the fluorescent signal on the cell surface is detected using a flow cytometer (BD FACS Celesta), obtaining and the median fluorescence intensity (MFI). A dose-response curve is generated, and the half-maximal effective concentration (EC50) is calculated using non-linear regression with GraphPad software. The results are shown in Table 1.

**Table1: Binding affinity to L1CAM on the surface of Hela cells of humanized anti-L1CAM antibody**

| Humanized antibody NO. | EC50(nM) |
|---|---|
| hz26A12C1H4L3 | 0.7378 |
| hz46F1D5H4L3 | 1.272 |
| hz49E10H1H5L6 | 0.4042 |
| hz49E10H1H5L7 | 0.3767 |
| hz103E9B3H5L5 | 1.682 |
| hz103E9B3H6L5 | 1.483 |

### Example 5: Conjugation of humanized anti-L1CAM monoclonal molecule with MMAE

10 mg/mL monoclonal antibody solution, 1 mmol/L diethylenetriaminepentaacetic acid (DTPA), and 200 µmol/L tris (2-carboxyethyl)-phosphine (TCEP) are mixed in PBS buffer and stirred and reacted at 37°C for 2 hours, the partially reduced antibody is obtained. The concentration of free thiol groups is determined using DTNB assay thereby the reduction degree of the antibody is determined.

The antibody reduced with TCEP can be directly used for subsequent conjugation. The 10 mM solution of maleimide-val-cit-PABC-MMAE (mc-vc-PABC-MMAE) drug is configured using 20% DMSO, and the drug is added slowly at a drug-to-antibody molar ratio of 8:1, stirred and reacted at room temperature for 3 hours, followed by Sephadex G-25 purification to remove residual unreacted drug and free small molecules, and the conjugation is assessed by SDS-PAGE electrophoresis, hydrophobic interaction high-performance liquid chromatography (HIC-HPLC), reverse-phase high-performance liquid chromatography (RP-HPLC), and size exclusion high-performance liquid chromatography (SEC-HPLC).

### Example 6: Determination of binding affinity to L1CAM on the surface of cells of humanized anti-L1CAM antibody-drug conjugate (anti-L1CAM ADC)

The experimental principle is as follows: HeLa cells (ATCC, CCL-2) express L1CAM protein on their surface. After incubating anti-L1CAM ADCs with cells, the binding ability to L1CAM on the cell surface of the antibody can be determined by flow cytometry, thereby to determine affinity to antigen of the antibody.

The experimental method is described as following:
The anti-L1CAM ADC is diluted with complete culture medium in a 4-fold serial dilution, with 10 concentration gradient points.

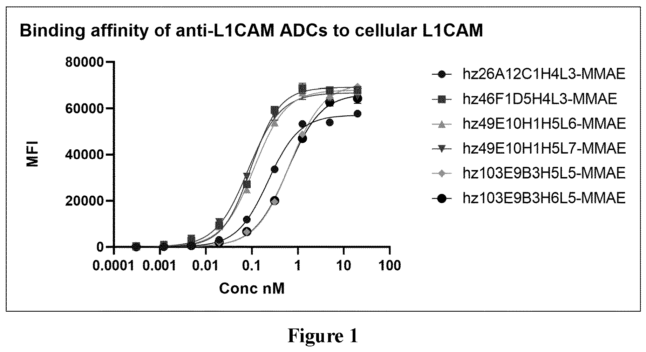

HeLa cells are cultured in DMEM complete medium (Corning, 10-013-CVR) containing 10% FBS (Gibco, 10099-141) and 100 U/mL penicillin-streptomycin mixture (Thermo Fisher, 15140122). When the cell coverage in the culture container reaches 80-90%, TrypLE (Thermofisher, 12604-013) is used to digest the HeLa cells from the culture container, and they are seeded in a 96-deepwell plate at a density of 3.7 × 10⁴ cells/ml, with 50,000 cells (1350µL complete medium) per well. Then, 150 µL of the ADC dilution is added to each well, mixed gently, and the 96-well plate is incubated at 4°C for 1 hour. Then the plate is centrifuged at 1,400 rpm for 4 minutes, the supernatant is removed, and 150 µL of PBS solution (Corning, 21-040-CVC) containing 2% FBS is added to each well, centrifuged at 1,400 rpm for 4 minutes, then washed twice. Then, 50 µL of Alexa Fluor 647-labeled goat anti-human fluorescent secondary antibody (JacksonImmuno, 109-605-088) diluted at 1:800 is added to each well, and the 96-well plate is incubated at 4°C for 0.5 hours. Cells are washed twice with PBS solution containing 2% FBS, and then resuspended in 120 µL of PBS, and the cell surface fluorescence signal is detected by using a flow cytometer (BD FACS Celesta), obtaining the median fluorescence intensity (MFI). A dose-response curve is generated, and the equilibrium dissociation constant (KD) is calculated using non-linear regression with GraphPad software. The results are shown in Table 2 and Figure 1.

**Table 2: Affinity equilibrium dissociation constant to L1CAM on the surface of HeLa cells of humanized anti-L1CAM antibody-drug conjugate (anti-L1CAM ADC)**

| anti-L1CAM ADC | KD (nM) |
|---|---|
| hz26A12C1H4L3-MMAE | 0.23 |
| hz46F1D5H4L3-MMAE | 0.097 |
| hz49E10H1H5L6-MMAE | 0.11 |
| hz49E10H1H5L7-MMAE | 0.084 |
| hz103E9B3H5L5-MMAE | 0.66 |
| hz103E9B3H6L5-MMAE | 0.60 |

### Example 7: Determination of the binding specificity to L1CAM on the surface of cells of humanized anti-L1CAM antibody-drug conjugate (anti-L1CAMADC)

The experimental principle is as follows: HeLa cells (ATCC, CCL-2) express high level of L1CAM protein on their surface, while HeLa L1CAM KO cells (Abcam, ab255401) exhibit significantly decreased expression level of L1CAM due to knockout of L1CAM gene. After incubating the anti-L1CAM ADCs with HeLa and HeLa KO cells, the binding ability to L1CAM on the surface of cells of the antibody can be detected using method of flow cytometry, thereby determining the binding specificity of anti-L1CAM ADC.

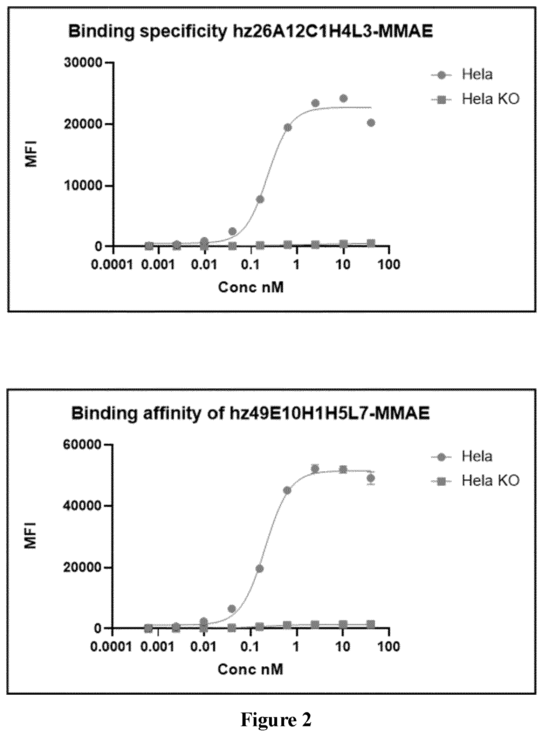

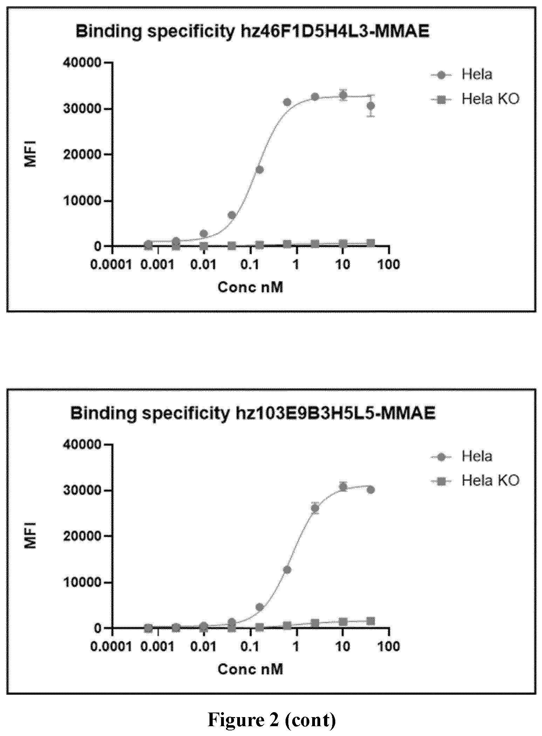

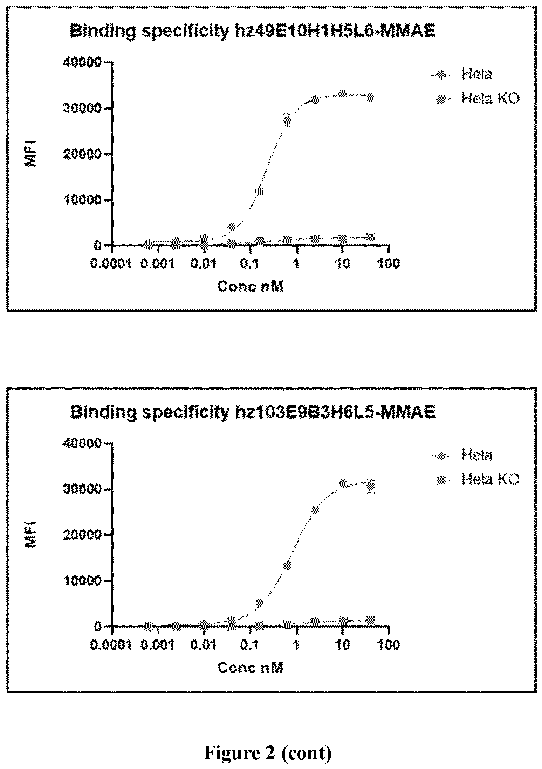

The experimental method is described as following:
HeLa cells and HeLa L1CAM-KO cells are cultured in DMEM complete medium (Corning, 10-013-CVR) containing 10% FBS (Gibco, 10099-141) and 100 U/mL penicillin-streptomycin mixture (Thermo Fisher, 15140122). When the cell coverage in the culture container reaches 80-90%, TrypLE (Thermofisher, 12604-013) is used to digest the cells from the culture container, and they are seeded in a 96-well plate (AXYGEN, P-96-450V-C) at a density of 5.5 × 10⁵ cells/ml, with 100,000 cells (90µL complete medium) per well, then, 10 µL of anti-L1CAMADC dilution is added to each well, mixed gently, and the 96-well plate is incubated at 4°C for 1 hour. Then, 150 µL of PBS (Corning, 21-040-CVC) containing 2% FBS is added to each well, centrifuged at 1,400 rpm for 4 minutes, the supernatant is removed, and washed once again. Subsequently, 50 µL of Alexa Fluor 647-labeled goat anti-human fluorescent secondary antibody (Jacksonimmuno, 109-605-088) diluted at 1:800 is added to each well, and the 96-well plate is incubated at 4°C for 0.5 hours. Cells are washed twice using PBS solution containing 2% FBS, then resuspended in 120 µL of PBS, and cell surface fluorescence signal is detected using flow cytometer (BD FACS Celesta), obtaining the median fluorescence intensity (MFI). A dose-response curve is generated using GraphPad software, and the experimental results are shown in Figure 2. The results indicate that the anti-L1CAM ADC possesses good binding specificity.

### Example 8: Determination of cell killing ability to Hela and Hela L1CAM KO cells of humanized anti-L1CAM antibody-drug conjugates (anti-L1CAM ADCs)

The experimental principle is as follows: HeLa cells (Cobioer Biosciences Co., LTD, CBP62032) express high level of L1CAM protein on their surface, while Hela L1CAM KO cells (Abcam, ab255401) have significantly decreased expression level of L1CAM due to knockout of L1CAM gene. Incubation of anti-L1CAM ADC with the cells leads to internalization of the antigen-antibody complexes, and then the ADC molecules release toxins inside the cell to kill tumor cells. The specific cytotoxicity of the ADC molecules against HeLa cells is determined by method of CellTiter-Glo (CTG) Luminescent Cell Viability Assay (Promega, G7573), and meanwhile the selectivity of the ADC molecules is determined by detecting the killing activity of the ADC molecules against Hela L1CAM KO cells.

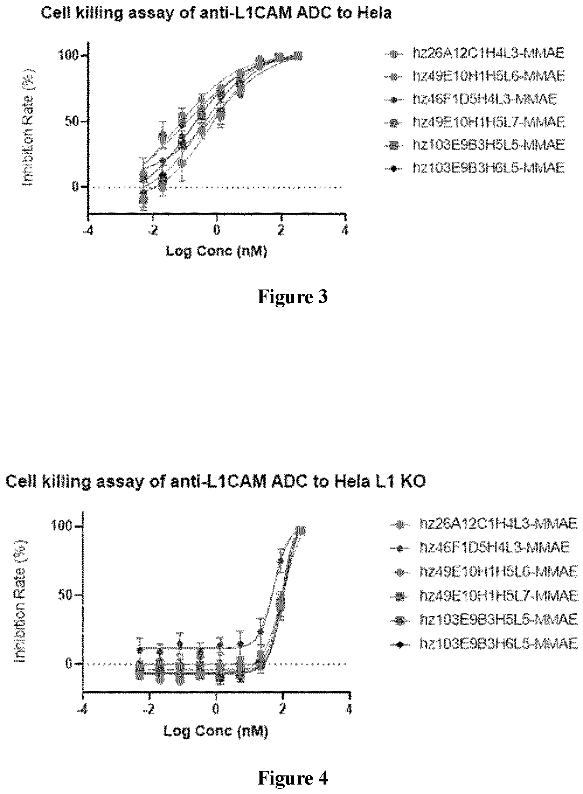

The experimental method is described as following:
HeLa and HeLa L1CAM-KO cells are cultured in DMEM complete medium (Corning, 10-013-CVR) containing 10% FBS (Gibco, 10099-141) and 100 U/mL penicillin-streptomycin mixture (Thermofisher, 15140122). When the cell coverage in the culture container reaches 80-90%, TrypLE (Thermofisher, 12604-013) is used to digest the HeLa and HeLa KO cells from the culture container, and they are seeded in a 96-well cell culture plate (Greiner, 655098) at a density of 3.3 × 10³ cells/mL, with 500 cells per well (145 µL complete medium),and the 96-well plate is incubated at 37°C overnight. The next day, the humanized anti-L1CAM antibody-MMAE conjugate (ADC) is diluted in complete medium in a 4-fold serial dilution, with 9 concentration gradient points. Then, 5 µL of dilution of the ADC molecule is added to each well in the 96-well cell culture plate, gently mixed, and the 96-well plate is incubated at 37°C for 6 days. Finally, 75 µL of CTG is added to each well, gently mixed, and the 96-well plate is incubated at room temperature for 10 minutes. Then the luminescence signal is detected using a multimode microplate reader (PerkinElmer Envision 2105). A dose-inhibition curve is generated, and the half-maximal inhibitory concentration (IC50) is calculated using non-linear regression with GraphPad software. The experimental results are shown in Table 3, Figure 3, and Figure 4.

**Table 3: Cell killing assay of humanized anti-L1CAM antibody-drug conjugates (anti-L1CAM ADCs) against HeLa and HeLa L1CAM KO cells**

| Cell killing activity of anti-L1CAM ADC (IC50 nM) | | |
|---|---|---|
| ADC | HeLa | HeLa L1 KO |
| hz26A12C1H4L3-MMAE | 0.635 | 100 |
| hz46F1D5H4L3-MMAE | 0.395 | 44 |
| hz49E10H1H5L6-MMAE | 0.061 | 95 |
| hz49E10H1H5L7-MMAE | 0.092 | 90 |
| hz103E9B3H5L5-MMAE | 0.32 | 91 |
| hz103E9B3H6L5-MMAE | 0.155 | 100 |

**Table 4: Amino acid sequence of complementarity-determining regions of the L1CAM antibodies of the present invention**

| Antibody NO. | Amino acid sequence | | | SEQ ID NO |
|---|---|---|---|---|
| hz26A12C1H4L 3 | Heavy chain complementarity determining region | HCDR1 | GYTFTNYW | 1 |
| | | HCDR2 | INPSTYYS | 2 |
| | | HCDR3 | ARRQFGLPLFDY | 3 |
| | Light chain complementarity determining region | LCDR1 | QDVGTA | 4 |
| | | LCDR2 | WAS | 5 |
| | | LCDR3 | QQYSNYPLT | 6 |
| hz46F1D5H4L3 | Heavy chain complementarity determining region | HCDR1 | GYTFTRYW | 7 |
| | | HCDR2 | INPSTGYT | 8 |
| | | HCDR3 | ARRHLGLPLFDY | 9 |
| | Light chain complementarity-det | LCDR1 | QDVGTA | 4 |
| | | LCDR2 | WAS | 5 |
| | ermining regions | LCDR3 | QQYSNYPLT | 6 |
| hz49E10H1H5L 6 | Heavy chain complementarity determining region | HCDR1 | GGSISSDYA | 10 |
| | | HCDR2 | ITYSGTT | 11 |
| | | HCDR3 | ARDHYGNYAYFDY | 12 |
| | Light chain complementarity determining region | LCDR1 | SSVSF | 13 |
| | | LCDR2 | ASS | 14 |
| | | LCDR3 | QQWSSNPST | 15 |
| hz49E10H1H5L 7 | Heavy chain complementarity determining region | HCDR1 | GGSISSDYA | 10 |
| | | HCDR2 | ITYSGTT | 11 |
| | | HCDR3 | ARDHYGNYAYFDY | 12 |
| | Light chain complementarity determining region | LCDR1 | SSVSF | 13 |
| | | LCDR2 | ASS | 14 |
| | | LCDR3 | QQWSSNPST | 15 |
| hz103E9B3H5L5 | Heavy chain complementarity determining region | HCDR1 | GYTFTSYT | 16 |
| | | HCDR2 | INPNYGG | 17 |
| | | HCDR3 | AREGATGTWYFDV | 18 |
| | Light chain complementarity determining region | LCDR1 | SSVSF | 13 |
| | | LCDR2 | LTS | 19 |
| | | LCDR3 | QQWRSNPPT | 20 |
| hz103E9B3H6L5 | Heavy chain complementarity determining region | HCDR1 | GYTFTSYT | 16 |
| | | HCDR2 | INPNYGG | 17 |
| | | HCDR3 | AREGATGTWYFDV | 18 |
| | Light chain complementarity determining region | LCDR1 | SSVSF | 13 |
| | | LCDR2 | LTS | 19 |
| | | LCDR3 | QQWRSNPPT | 20 |

**Table 5: Amino acid sequence of variable regions of the humanized anti-L1CAM monoclonal antibodies of the present Invention**

| Antibody NO. | SEQ ID NO: | | Amino acid sequence |
|---|---|---|---|
| hz26A12C1H4L3 | VH | 21 | DIQMTQSPSSLSASVGDRVTITCSASSSVSFMFWYQ QKPGKAPKPLIYLTSNLASGVPSRFSGSGSGTDFTLT ISSLQPEDFATYYCQQWRSNPPTFGGGTKVEIK |
| | VL | 22 | DIQMTQSPSSLSASVGDRVTITCRASQDVGTAVAWY QQKPGKVPKLLIYWASTLHSGVPSRFSGSGSGTDFT LTISSLQPEDVATYFCQQYSNYPLTFGGGTKVEIK |
| hz46F1D5H4L3 | VH | 23 | QVQLVQSGAEVKKPGSSVKVSCKASGYTFTRYWIQ WVRQAPGQGLEWMGYINPSTGYTDYNQKFQGRV TITADKSTSTAYMELSSLRSEDTAVYYCARRHLGLP LFDYWGQGTTVTVSS |
| | VL | 24 | DIQMTQSPSSVSASVGDRVTITCRASQDVGTAVAWY QQKPGKAPKLLIYWASSLHSGVPSRFSGSGSGTDFT LTISSLQPEDFATYFCQQYSNYPLTFGGGTKVEIK |
| hz49E10H1H5L6 | VH | 25 | DVQLQESGPGLVKPSQTLSLTCTVSGGSISSDYAWN WIRQPPGKGLEWMGYITYSGTTTYNPSLKSRITISR DTSKNQFSLKLSSVTAADTAVYYCARDHYGNYAYF DYWGQGTTVTVSS |
| | VL | 26 | DIQMTQSPSSLSASVGDRVTITCRASSSVSFMHWYQ QKPGKAPKPWIYASSNLVSGVPSRFSGSGSGTDFTL TISSLQPEDFATYYCQQWSSNPSTFGGGTKVEIK |
| hz49E10H1H5L7 | VH | 27 | DVQLQESGPGLVKPSQTLSLTCTVSGGSISSDYAWN WIRQPPGKGLEWMGYITYSGTTTYNPSLKSRITISR DTSKNQFSLKLSSVTAADTAVYYCARDHYGNYAYF DYWGQGTTVTVSS |
| | VL | 28 | QIQMTQSPSSLSASVGDRVTITCRASSSVSFMHWYQ QKPGKAPKPWIYASSNLVSGVPSRFSGSGSGTDYTL TISSLQPEDFATYYCQQWSSNPSTFGGGTKVEIK |
| hz103E9B3H5L5 | VH | 29 | EVQLVQSGAEVKKPGASVKVSCKASGYTFTSYTM HWVRQAPGQGLEWMGDINPNYGGTSYNEKFKDR VTMTTDTSTSTAYMELRSLRSDDTAVYYCAREGAT GTWYFDVWGQGTTVTVSS |
| | VL | 30 | DIQMTQSPSSLSASVGDRVTITCSASSSVSFMFWYQ QKPGKAPKPLIYLTSNLASGVPSRFSGSGSGTDFTLT ISSLQPEDFATYYCQQWRSNPPTFGGGTKVEIK |
| hz103E9B3H6L5 | VH | 31 | EVQLVQSGAEVKKPGASVKVSCKASGYTFTSYTM HWVRQAPGQGLEWMGDINPNYGGTSYNEKFKDR ATMTTDTSTSTAYMELRSLRSDDTAVYYCAREGAT |
| | | | GTWYFDVWGQGTTVTVSS |
| | VL | 32 | DIQMTQSPSSLSASVGDRVTITCSASSSVSFMFWYQ QKPGKAPKPLIYLTSNLASGVPSRFSGSGSGTDFTLT ISSLQPEDFATYYCQQWRSNPPTFGGGTKVEIK |

**Table 6: Amino acid sequence of the humanized anti-L1CAM monoclonal antibodies of the present invention**

| Antibody NO. | SEQ ID NO: | | Amino acid sequence |
|---|---|---|---|
| hz26A12C1H4L3 | Heavy chain | 33 | QVQLVQSGAEVKKPGSSVKVSCKASGYTFTNYWIQ WVRQAPGQGLEWIGYINPSTYYSDYNQKFQGRVTL TADKSTSTAYMELSSLRSEDTAVYYCARRQFGLPLF DYWGQGTTVTVSSASTKGPSVFPLAPSSKSTSGGT AALGCLVKDYFPEPVTVSWNSGALTSGVHTFPAVL QSSGLYSLSSVVTVPSSSLGTQTYICNVNHKPSNTK VDKKVEPKSCDKTHTCPPCPAPELLGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSREEMTKNQVSLTCLVKGFYPSDIAVEWESNGQP ENNYKTTPPVLDSDGSFFLYSKLTVDKSRWQQGNV FSCSVMHEALHNHYTQKSLSLSPGK |
| | Light chain | 34 | DIQMTQSPSSLSASVGDRVTITCRASQDVGTAVAWY QQKPGKVPKLLIYWASTLHSGVPSRFSGSGSGTDFT LTISSLQPEDVATYFCQQYSNYPLTFGGGTKVEIKRT VAAPSVFIFPPSDEQLKSGTASVVCLLNNFYPREAK VQWKVDNALQSGNSQESVTEQDSKDSTYSLSSTLT LSKADYEKHKVYACEVTHQGLSSPVTKSFNRGEC |
| hz46F1D5H4L3 | Heavy chain | 35 | QVQLVQSGAEVKKPGSSVKVSCKASGYTFTRYWIQ WVRQAPGQGLEWMGYINPSTGYTDYNQKFQGRV TITADKSTSTAYMELSSLRSEDTAVYYCARRHLGLP LFDYWGQGTTVTVSS ASTKGPSVFPLAPSSKSTSGGTAALGCLVKDYFPEP VTVSWNSGALTSGVHTFPAVLQSSGLYSLSSVVTVP |
| | | | SSSLGTQTYICNVNHKPSNTKVDKKVEPKSCDKTH TCPPCPAPELLGGPSVFLFPPKPKDTLMISRTPEVTC VVVDVSHEDPEVKFNWYVDGVEVHNAKTKPREE QYNSTYRVVSVLTVLHQDWLNGKEYKCKVSNKAL PAPIEKTISKAKGQPREPQVYTLPPSREEMTKNQVSL TCLVKGFYPSDIAVEWESNGQPENNYKTTPPVLDSD GSFFLYSKLTVDKSRWQQGNVFSCSVMHEALHNH YTQKSLSLSPGK |
| | Light chain | 36 | DIQMTQSPSSVSASVGDRVTITCRASQDVGTAVAWY QQKPGKAPKLLIYWASSLHSGVPSRFSGSGSGTDFT LTISSLQPEDFATYFCQQYSNYPLTFGGGTKVEIKRT VAAPSVFIFPPSDEQLKSGTASVVCLLNNFYPREAK VQWKVDNALQSGNSQESVTEQDSKDSTYSLSSTLT LSKADYEKHKVYACEVTHQGLSSPVTKSFNRGEC |
| hz49E10H1H5L6 | Heavy chain | 37 | DVQLQESGPGLVKPSQTLSLTCTVSGGSISSDYAWN WIRQPPGKGLEWMGYITYSGTTTYNPSLKSRITISR DTSKNQFSLKLSSVTAADTAVYYCARDHYGNYAYF DYWGQGTTVTVSSASTKGPSVFPLAPSSKSTSGGT AALGCLVKDYFPEPVTVSWNSGALTSGVHTFPAVL QSSGLYSLSSVVTVPSSSLGTQTYICNVNHKPSNTK VDKKVEPKSCDKTHTCPPCPAPELLGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSREEMTKNQVSLTCLVKGFYPSDIAVEWESNGQP ENNYKTTPPVLDSDGSFFLYSKLTVDKSRWQQGNV FSCSVMHEALHNHYTQKSLSLSPGK |
| | Light chain | 38 | DIQMTQSPSSLSASVGDRVTITCRASSSVSFMHWYQ QKPGKAPKPWIYASSNLVSGVPSRFSGSGSGTDFTL TISSLQPEDFATYYCQQWSSNPSTFGGGTKVEIKRTV AAPSVFIFPPSDEQLKSGTASVVCLLNNFYPREAKV QWKVDNALQSGNSQESVTEQDSKDSTYSLSSTLTL SKADYEKHKVYACEVTHQGLSSPVTKSFNRGEC |
| hz49E10H1H5L7 | Heavy | 39 | DVQLQESGPGLVKPSQTLSLTCTVSGGSISSDYAWN |
| | chain | | WIRQPPGKGLEWMGYITYSGTTTYNPSLKSRITISR DTSKNQFSLKLSSVTAADTAVYYCARDHYGNYAYF DYWGQGTTVTVSSASTKGPSVFPLAPSSKSTSGGT AALGCLVKDYFPEPVTVSWNSGALTSGVHTFPAVL QSSGLYSLSSVVTVPSSSLGTQTYICNVNHKPSNTK VDKKVEPKSCDKTHTCPPCPAPELLGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSREEMTKNQVSLTCLVKGFYPSDIAVEWESNGQP ENNYKTTPPVLDSDGSFFLYSKLTVDKSRWQQGNV FSCSVMHEALHNHYTQKSLSLSPGK |
| | Light chain | 40 | QIQMTQSPSSLSASVGDRVTITCRASSSVSFMHWYQ QKPGKAPKPWIYASSNLVSGVPSRFSGSGSGTDYTL TISSLQPEDFATYYCQQWSSNPSTFGGGTKVEIKRTV AAPSVFIFPPSDEQLKSGTASVVCLLNNFYPREAKV QWKVDNALQSGNSQESVTEQDSKDSTYSLSSTLTL SKADYEKHKVYACEVTHQGLSSPVTKSFNRGEC |
| hz103E9B3H5L5 | Heavy chain | 41 | EVQLVQSGAEVKKPGASVKVSCKASGYTFTSYTM HWVRQAPGQGLEWMGDINPNYGGTSYNEKFKDR VTMTTDTSTSTAYMELRSLRSDDTAVYYCAREGAT GTWYFDVWGQGTTVTVSSASTKGPSVFPLAPSSKS TSGGTAALGCLVKDYFPEPVTVSWNSGALTSGVHT FPAVLQSSGLYSLSSVVTVPSSSLGTQTYICNVNHKP SNTKVDKKVEPKSCDKTHTCPPCPAPELLGGPSVFL FPPKPKDTLMISRTPEVTCVVVDVSHEDPEVKFNW YVDGVEVHNAKTKPREEQYNSTYRVVSVLTVLHQ DWLNGKEYKCKVSNKALPAPIEKTISKAKGQPREP QVYTLPPSREEMTKNQVSLTCLVKGFYPSDIAVEWE SNGQPENNYKTTPPVLDSDGSFFLYSKLTVDKSRW QQGNVFSCSVMHEALHNHYTQKSLSLSPGK |
| | Light chain | 42 | DIQMTQSPSSLSASVGDRVTITCSASSSVSFMFWYQ QKPGKAPKPLIYLTSNLASGVPSRFSGSGSGTDFTLT ISSLQPEDFATYYCQQWRSNPPTFGGGTKVEIKRTV |
| | | | AAPSVFIFPPSDEQLKSGTASVVCLLNNFYPREAKV QWKVDNALQSGNSQESVTEQDSKDSTYSLSSTLTL SKADYEKHKVYACEVTHQGLSSPVTKSFNRGEC |
| hz103E9B3H6L5 | Heavy chain | 43 | EVQLVQSGAEVKKPGASVKVSCKASGYTFTSYTM HWVRQAPGQGLEWMGDINPNYGGTSYNEKFKDR ATMTTDTSTSTAYMELRSLRSDDTAVYYCAREGAT GTWYFDVWGQGTTVTVSSASTKGPSVFPLAPSSKS TSGGTAALGCLVKDYFPEPVTVSWNSGALTSGVHT FPAVLQSSGLYSLSSVVTVPSSSLGTQTYICNVNHKP SNTKVDKKVEPKSCDKTHTCPPCPAPELLGGPSVFL FPPKPKDTLMISRTPEVTCVVVDVSHEDPEVKFNW YVDGVEVHNAKTKPREEQYNSTYRVVSVLTVLHQ DWLNGKEYKCKVSNKALPAPIEKTISKAKGQPREP QVYTLPPSREEMTKNQVSLTCLVKGFYPSDIAVEWE SNGQPENNYKTTPPVLDSDGSFFLYSKLTVDKSRW QQGNVFSCSVMHEALHNHYTQKSLSLSPGK |
| | Light chain | 44 | DIQMTQSPSSLSASVGDRVTITCSASSSVSFMFWYQ QKPGKAPKPLIYLTSNLASGVPSRFSGSGSGTDFTLT ISSLQPEDFATYYCQQWRSNPPTFGGGTKVEIKRTV AAPSVFIFPPSDEQLKSGTASVVCLLNNFYPREAKV QWKVDNALQSGNSQESVTEQDSKDSTYSLSSTLTL SKADYEKHKVYACEVTHQGLSSPVTKSFNRGEC |

**Table 7: Nucleotide sequences encoding the humanized anti-L1CAM monoclonal antibodies of the present invention**

| Antibody NO. | SEQ ID NO: | | Nucleotide Sequence |
|---|---|---|---|
| hz26A12C1H4L3 | Heavy chain | 45 | CAGGTCCAACTCGTCCAAAGCGGCGCAGAAGTC AAAAAACCCGGCAGCTCAGTCAAAGTTAGCTGC AAGGCCAGCGGCTACACCTTCACAAACTACTGGA TTCAATGGGTGAGACAAGCCCCAGGCCAAGGGC TCGAATGGATAGGCTACATCAACCCCAGCACCTA CTATAGCGACTACAACCAGAAATTCCAGGGCAGA GTCACCCTGACCGCCGACAAAAGCACCAGCACC |
| | | | GCTTACATGGAACTGTCCAGCCTGAGAAGCGAGG ACACCGCCGTGTACTACTGCGCCAGAAGACAATT CGGACTGCCCCTGTTCGATTACTGGGGCCAGGGA ACCACCGTGACCGTGAGCAGC GCTAGCACCAAGGGCCCATCGGTCTTCCCCCTGG CACCCTCCTCCAAGAGCACCTCTGGGGGCACAGC GGCCCTGGGCTGCCTGGTCAAGGACTACTTCCCC GAACCGGTGACGGTGTCGTGGAACTCAGGCGCC CTGACCAGCGGCGTGCACACCTTCCCGGCTGTCC TACAGTCCTCAGGACTCTACTCCCTCAGCAGCGT GGTGACCGTGCCCTCCAGCAGCTTGGGCACCCAG ACCTACATCTGCAACGTGAATCACAAGCCCAGCA ACACCAAGGTGGACAAGAAAGTTGAGCCCAAAT CTTGTGACAAAACTCACACATGCCCACCGTGCCC AGCACCTGAACTCCTGGGGGGACCGTCAGTCTTC CTCTTCCCCCCAAAACCCAAGGACACCCTCATGA TCTCCCGGACCCCCGAGGTCACATGCGTGGTGGT GGACGTGAGCCACGAAGACCCTGAGGTCAAGTT CAACTGGTACGTGGACGGCGTGGAGGTGCATAAT GCCAAGACAAAGCCGCGGGAGGAGCAGTACAAC AGCACGTACCGTGTGGTCAGCGTCCTCACCGTCC TGCACCAGGACTGGCTGAATGGCAAGGAGTACA AGTGCAAGGTCTCCAACAAAGCCCTCCCAGCCCC CATCGAGAAAACCATCTCCAAAGCCAAAGGGCA GCCCCGAGAACCACAGGTGTACACCCTGCCCCCA TCCCGGGAGGAGATGACCAAGAACCAGGTCAGC CTGACCTGCCTGGTCAAAGGCTTCTATCCCAGCG ACATCGCCGTGGAGTGGGAGAGCAATGGGCAGC CGGAGAACAACTACAAGACCACGCCTCCCGTGCT GGACTCCGACGGCTCCTTCTTCCTCTACAGCAAG CTCACCGTGGACAAGAGCAGGTGGCAGCAGGGG AACGTCTTCTCATGCTCCGTGATGCATGAGGCTCT GCACAACCACTACACGCAGAAGAGCCTCTCCCTG TCTCCGGGTAAA |
| | Light chain | 46 | GACATTCAGATGACCCAGAGCCCCAGCAGCCTG[?] GCGCCAGCGTGGGAGACAGAGTGACCATAACCT[?] GCAGAGCCAGCCAAGACGTTGGAACCGCCGTG[?] CCTGGTATCAGCAGAAACCCGGAAAGGTGCCCA AACTGCTGATCTACTGGGCCAGCACCCTGCATT[?] CGGAGTGCCCAGCAGATTCAGCGGAAGCGGAA[?] CGGGACAGACTTCACCCTGACCATCAGCAGCCT[?] CAACCAGAGGACGTCGCCACCTACTTCTGCCAG[?] AGTACAGCAACTACCCCCTGACCTTCGGAGGCGG CACAAAAGTCGAAATCAAG CGTACGGTGGCTGCACCATCTGTCTTCATCTTCCC GCCATCTGATGAGCAGTTGAAATCTGGAACTGCC TCTGTTGTGTGCCTGCTGAATAACTTCTATCCCAG AGAGGCCAAAGTACAGTGGAAGGTGGATAACGC CCTCCAATCGGGTAACTCCCAGGAGAGTGTCACA GAGCAGGACAGCAAGGACAGCACCTACAGCCTC AGCAGCACCCTGACGCTGAGCAAAGCAGACTAC GAGAAACACAAAGTCTACGCCTGCGAAGTCACC CATCAGGGCCTGAGTTCGCCCGTCACAAAGAGCT TCAACAGGGGAGAGTGT |
| hz46F1D5H4L3 | Heavy chain | 47 | CAGGTCCAACTCGTCCAAAGCGGCGCAGAAGTC AAAAAACCCGGCAGCTCAGTCAAAGTTAGCTGC AAGGCCAGCGGCTACACCTTCACAAGATACTGGA TTCAATGGGTGAGACAAGCCCCAGGCCAAGGGC TCGAATGGATGGGATACATCAACCCCTCAACCGG CTACACCGACTACAACCAGAAGTTCCAGGGCAGA GTGACCATCACCGCCGACAAAAGCACAAGCACC GCCTACATGGAGCTGTCCAGCCTGCGCAGCGAGG ACACCGCCGTCTACTATTGCGCCAGAAGACACCT CGGCCTGCCCCTGTTCGATTACTGGGGCCAAGGA ACCACAGTGACCGTGAGTAGC GCTAGCACCAAGGGCCCATCGGTCTTCCCCCTGG CACCCTCCTCCAAGAGCACCTCTGGGGGCACAGC GGCCCTGGGCTGCCTGGTCAAGGACTACTTCCCC |
| | | | GAACCGGTGACGGTGTCGTGGAACTCAGGCGCC CTGACCAGCGGCGTGCACACCTTCCCGGCTGTCC TACAGTCCTCAGGACTCTACTCCCTCAGCAGCGT GGTGACCGTGCCCTCCAGCAGCTTGGGCACCCAG ACCTACATCTGCAACGTGAATCACAAGCCCAGCA ACACCAAGGTGGACAAGAAAGTTGAGCCCAAAT CTTGTGACAAAACTCACACATGCCCACCGTGCCC AGCACCTGAACTCCTGGGGGGACCGTCAGTCTTC CTCTTCCCCCCAAAACCCAAGGACACCCTCATGA TCTCCCGGACCCCCGAGGTCACATGCGTGGTGGT GGACGTGAGCCACGAAGACCCTGAGGTCAAGTT CAACTGGTACGTGGACGGCGTGGAGGTGCATAAT GCCAAGACAAAGCCGCGGGAGGAGCAGTACAAC AGCACGTACCGTGTGGTCAGCGTCCTCACCGTCC TGCACCAGGACTGGCTGAATGGCAAGGAGTACA AGTGCAAGGTCTCCAACAAAGCCCTCCCAGCCCC CATCGAGAAAACCATCTCCAAAGCCAAAGGGCA GCCCCGAGAACCACAGGTGTACACCCTGCCCCCA TCCCGGGAGGAGATGACCAAGAACCAGGTCAGC CTGACCTGCCTGGTCAAAGGCTTCTATCCCAGCG ACATCGCCGTGGAGTGGGAGAGCAATGGGCAGC CGGAGAACAACTACAAGACCACGCCTCCCGTGCT GGACTCCGACGGCTCCTTCTTCCTCTACAGCAAG CTCACCGTGGACAAGAGCAGGTGGCAGCAGGGG AACGTCTTCTCATGCTCCGTGATGCATGAGGCTCT GCACAACCACTACACGCAGAAGAGCCTCTCCCTG TCTCCGGGTAAA |
| | Light chain | 48 | GACATTCAGATGACCCAGAGCCCCAGCAGCGTGA GCGCCAGCGTGGGAGACAGAGTGACCATAACCT GCAGAGCCAGCCAAGACGTTGGAACCGCCGTGG CCTGGTATCAGCAGAAACCCGGAAAGGCCCCCA AACTGCTCATCTACTGGGCAAGCAGCCTGCACAG CGGAGTGCCCAGCAGATTTAGCGGAAGCGGAAG CGGGACAGACTTTACCCTGACCATCAGCAGCCTC |
| | | | CAACCTGAGGATTTCGCCACCTACTTCTGCCAGC AATACAGCAACTACCCACTGACCTTCGGAGGCGG CACAAAAGTCGAGATCAAG CGTACGGTGGCTGCACCATCTGTCTTCATCTTCCC GCCATCTGATGAGCAGTTGAAATCTGGAACTGCC TCTGTTGTGTGCCTGCTGAATAACTTCTATCCCAG AGAGGCCAAAGTACAGTGGAAGGTGGATAACGC CCTCCAATCGGGTAACTCCCAGGAGAGTGTCACA GAGCAGGACAGCAAGGACAGCACCTACAGCCTC AGCAGCACCCTGACGCTGAGCAAAGCAGACTAC GAGAAACACAAAGTCTACGCCTGCGAAGTCACC CATCAGGGCCTGAGTTCGCCCGTCACAAAGAGCT TCAACAGGGGAGAGTGT |
| hz49E10H1H5L6 | Heavy chain | 49 | GACGTGCAGCTGCAAGAGAGCGGACCCGGACTG GTGAAGCCCAGCCAAACACTGAGCCTGACATGC ACAGTGAGCGGAGGAAGCATAAGCAGCGACTAC GCCTGGAACTGGATTAGACAGCCCCCCGGCAAGG GCCTGGAGTGGATGGGATATATCACCTACAGCGG CACCACCACCTACAATCCCAGCCTGAAAAGCAGA ATCACCATCTCCAGGGACACCAGCAAGAACCAGT TCAGCCTCAAGCTGAGCAGCGTGACCGCCGCCG ACACCGCAGTCTACTACTGCGCCCGAGACCACTA CGGAAACTACGCCTACTTCGACTACTGGGGCCAG GGCACCACCGTGACCGTGTCCAGC GCTAGCACCAAGGGCCCATCGGTCTTCCCCCTGG CACCCTCCTCCAAGAGCACCTCTGGGGGCACAGC GGCCCTGGGCTGCCTGGTCAAGGACTACTTCCCC GAACCGGTGACGGTGTCGTGGAACTCAGGCGCC CTGACCAGCGGCGTGCACACCTTCCCGGCTGTCC TACAGTCCTCAGGACTCTACTCCCTCAGCAGCGT GGTGACCGTGCCCTCCAGCAGCTTGGGCACCCAG ACCTACATCTGCAACGTGAATCACAAGCCCAGCA ACACCAAGGTGGACAAGAAAGTTGAGCCCAAAT CTTGTGACAAAACTCACACATGCCCACCGTGCCC |
| | | | AGCACCTGAACTCCTGGGGGGACCGTCAGTCTTC CTCTTCCCCCCAAAACCCAAGGACACCCTCATGA TCTCCCGGACCCCCGAGGTCACATGCGTGGTGGT GGACGTGAGCCACGAAGACCCTGAGGTCAAGTT CAACTGGTACGTGGACGGCGTGGAGGTGCATAAT GCCAAGACAAAGCCGCGGGAGGAGCAGTACAAC AGCACGTACCGTGTGGTCAGCGTCCTCACCGTCC TGCACCAGGACTGGCTGAATGGCAAGGAGTACA AGTGCAAGGTCTCCAACAAAGCCCTCCCAGCCCC CATCGAGAAAACCATCTCCAAAGCCAAAGGGCA GCCCCGAGAACCACAGGTGTACACCCTGCCCCCA TCCCGGGAGGAGATGACCAAGAACCAGGTCAGC CTGACCTGCCTGGTCAAAGGCTTCTATCCCAGCG ACATCGCCGTGGAGTGGGAGAGCAATGGGCAGC CGGAGAACAACTACAAGACCACGCCTCCCGTGCT GGACTCCGACGGCTCCTTCTTCCTCTACAGCAAG CTCACCGTGGACAAGAGCAGGTGGCAGCAGGGG AACGTCTTCTCATGCTCCGTGATGCATGAGGCTCT GCACAACCACTACACGCAGAAGAGCCTCTCCCTG TCTCCGGGTAAA |
| | Light chain | 50 | GACATTCAGATGACCCAGAGCCCCAGCAGCCTGA GCGCCAGCGTGGGAGACAGAGTGACCATAACCT GCAGAGCCAGCAGCAGCGTTAGCTTCATGCACTG GTACCAGCAGAAACCCGGCAAGGCCCCCAAACC CTGGATCTACGCCTCCAGCAACCTGGTGAGCGGA GTGCCAAGCAGATTCAGCGGCAGCGGCAGCGGA ACCGACTTCACCCTCACTATCAGCAGCCTCCAAC CCGAAGACTTCGCCACCTACTACTGCCAGCAGTG GAGCTCCAATCCCAGCACCTTCGGCGGCGGCACA AAGGTGGAGATCAAA CGTACGGTGGCTGCACCATCTGTCTTCATCTTCCC GCCATCTGATGAGCAGTTGAAATCTGGAACTGCC TCTGTTGTGTGCCTGCTGAATAACTTCTATCCCAG AGAGGCCAAAGTACAGTGGAAGGTGGATAACGC |
| | | | CCTCCAATCGGGTAACTCCCAGGAGAGTGTCACA GAGCAGGACAGCAAGGACAGCACCTACAGCCTC AGCAGCACCCTGACGCTGAGCAAAGCAGACTAC GAGAAACACAAAGTCTACGCCTGCGAAGTCACC CATCAGGGCCTGAGTTCGCCCGTCACAAAGAGCT TCAACAGGGGAGAGTGT |
| hz49E10H1H5L7 | Heavy chain | 51 | GACGTGCAGCTGCAAGAGAGCGGACCCGGACTG GTGAAGCCCAGCCAAACACTGAGCCTGACATGC ACAGTGAGCGGAGGAAGCATAAGCAGCGACTAC GCCTGGAACTGGATTAGACAGCCCCCCGGCAAGG GCCTGGAGTGGATGGGATATATCACCTACAGCGG CACCACCACCTACAATCCCAGCCTGAAAAGCAGA ATCACCATCTCCAGGGACACCAGCAAGAACCAGT TCAGCCTCAAGCTGAGCAGCGTGACCGCCGCCG ACACCGCAGTCTACTACTGCGCCCGAGACCACTA CGGAAACTACGCCTACTTCGACTACTGGGGCCAG GGCACCACCGTGACCGTGTCCAGC GCTAGCACCAAGGGCCCATCGGTCTTCCCCCTGG CACCCTCCTCCAAGAGCACCTCTGGGGGCACAGC GGCCCTGGGCTGCCTGGTCAAGGACTACTTCCCC GAACCGGTGACGGTGTCGTGGAACTCAGGCGCC CTGACCAGCGGCGTGCACACCTTCCCGGCTGTCC TACAGTCCTCAGGACTCTACTCCCTCAGCAGCGT GGTGACCGTGCCCTCCAGCAGCTTGGGCACCCAG ACCTACATCTGCAACGTGAATCACAAGCCCAGCA ACACCAAGGTGGACAAGAAAGTTGAGCCCAAAT CTTGTGACAAAACTCACACATGCCCACCGTGCCC AGCACCTGAACTCCTGGGGGGACCGTCAGTCTTC CTCTTCCCCCCAAAACCCAAGGACACCCTCATGA TCTCCCGGACCCCCGAGGTCACATGCGTGGTGGT GGACGTGAGCCACGAAGACCCTGAGGTCAAGTT CAACTGGTACGTGGACGGCGTGGAGGTGCATAAT GCCAAGACAAAGCCGCGGGAGGAGCAGTACAAC AGCACGTACCGTGTGGTCAGCGTCCTCACCGTCC |
| | | | TGCACCAGGACTGGCTGAATGGCAAGGAGTACA AGTGCAAGGTCTCCAACAAAGCCCTCCCAGCCCC CATCGAGAAAACCATCTCCAAAGCCAAAGGGCA GCCCCGAGAACCACAGGTGTACACCCTGCCCCCA TCCCGGGAGGAGATGACCAAGAACCAGGTCAGC CTGACCTGCCTGGTCAAAGGCTTCTATCCCAGCG ACATCGCCGTGGAGTGGGAGAGCAATGGGCAGC CGGAGAACAACTACAAGACCACGCCTCCCGTGCT GGACTCCGACGGCTCCTTCTTCCTCTACAGCAAG CTCACCGTGGACAAGAGCAGGTGGCAGCAGGGG AACGTCTTCTCATGCTCCGTGATGCATGAGGCTCT GCACAACCACTACACGCAGAAGAGCCTCTCCCTG TCTCCGGGTAAA |
| | Light chain | 52 | CAGATCCAAATGACCCAAAGCCCCAGCAGCCTCT CCGCCAGCGTCGGAGACAGAGTCACCATCACCTG CAGAGCCAGCAGCAGCGTCTCCTTCATGCACTGG TACCAACAGAAACCCGGCAAGGCCCCCAAGCCC TGGATCTACGCCTCCTCCAATCTGGTGTCAGGAGT GCCCAGCAGATTCAGCGGCAGCGGCAGCGGAAC CGACTACACCCTCACTATCAGCAGCCTCCAACCT GAAGATTTCGCCACCTATTACTGCCAACAGTGGA GCAGCAACCCCAGCACCTTCGGAGGCGGTACCA AGGTCGAAATCAAA CGTACGGTGGCTGCACCATCTGTCTTCATCTTCCC GCCATCTGATGAGCAGTTGAAATCTGGAACTGCC TCTGTTGTGTGCCTGCTGAATAACTTCTATCCCAG AGAGGCCAAAGTACAGTGGAAGGTGGATAACGC CCTCCAATCGGGTAACTCCCAGGAGAGTGTCACA GAGCAGGACAGCAAGGACAGCACCTACAGCCTC AGCAGCACCCTGACGCTGAGCAAAGCAGACTAC GAGAAACACAAAGTCTACGCCTGCGAAGTCACC CATCAGGGCCTGAGTTCGCCCGTCACAAAGAGCT TCAACAGGGGAGAGTGT |
| hz103E9B3H5L5 | Heavy | 53 | GAGGTGCAGCTGGTGCAGAGCGGAGCAGAGGTG |
| | chain | | AAGAAGCCAGGGGCCAGCGTGAAGGTGAGCTGT AAGGCTAGTGGGTACACATTTACAAGCTACACAA TGCACTGGGTGAGACAGGCCCCAGGCCAAGGAC TGGAGTGGATGGGAGACATAAACCCCAATTACGG AGGAACAAGCTACAACGAAAAATTCAAAGACAG AGTGACCATGACCACCGACACAAGCACAAGCAC CGCCTACATGGAGCTGAGAAGTCTGAGATCAGAC GACACCGCCGTCTACTACTGCGCCAGAGAGGGAG CCACCGGGACCTGGTACTTCGACGTGTGGGGCCA GGGAACAACAGTGACCGTGAGCAGC GCTAGCACCAAGGGCCCATCGGTCTTCCCCCTGG CACCCTCCTCCAAGAGCACCTCTGGGGGCACAGC GGCCCTGGGCTGCCTGGTCAAGGACTACTTCCCC GAACCGGTGACGGTGTCGTGGAACTCAGGCGCC CTGACCAGCGGCGTGCACACCTTCCCGGCTGTCC TACAGTCCTCAGGACTCTACTCCCTCAGCAGCGT GGTGACCGTGCCCTCCAGCAGCTTGGGCACCCAG ACCTACATCTGCAACGTGAATCACAAGCCCAGCA ACACCAAGGTGGACAAGAAAGTTGAGCCCAAAT CTTGTGACAAAACTCACACATGCCCACCGTGCCC AGCACCTGAACTCCTGGGGGGACCGTCAGTCTTC CTCTTCCCCCCAAAACCCAAGGACACCCTCATGA TCTCCCGGACCCCCGAGGTCACATGCGTGGTGGT GGACGTGAGCCACGAAGACCCTGAGGTCAAGTT CAACTGGTACGTGGACGGCGTGGAGGTGCATAAT GCCAAGACAAAGCCGCGGGAGGAGCAGTACAAC AGCACGTACCGTGTGGTCAGCGTCCTCACCGTCC TGCACCAGGACTGGCTGAATGGCAAGGAGTACA AGTGCAAGGTCTCCAACAAAGCCCTCCCAGCCCC CATCGAGAAAACCATCTCCAAAGCCAAAGGGCA GCCCCGAGAACCACAGGTGTACACCCTGCCCCCA TCCCGGGAGGAGATGACCAAGAACCAGGTCAGC CTGACCTGCCTGGTCAAAGGCTTCTATCCCAGCG ACATCGCCGTGGAGTGGGAGAGCAATGGGCAGC |
| | | | CGGAGAACAACTACAAGACCACGCCTCCCGTGCT GGACTCCGACGGCTCCTTCTTCCTCTACAGCAAG CTCACCGTGGACAAGAGCAGGTGGCAGCAGGGG AACGTCTTCTCATGCTCCGTGATGCATGAGGCTCT GCACAACCACTACACGCAGAAGAGCCTCTCCCTG TCTCCGGGTAAA |
| | Light chain | 54 | GACATTCAGATGACCCAGAGCCCCAGCAGCCTGA GCGCCAGCGTGGGAGACAGAGTGACCATAACCT GCAGCGCCAGCTCCAGCGTGAGCTTCATGTTTTG GTACCAGCAGAAACCCGGCAAGGCCCCCAAGCC CCTGATATACCTGACCAGCAACCTGGCCTCTGGA GTGCCCAGCCGGTTCTCCGGAAGCGGCTCTGGAA CTGACTTCACCCTCACCATCTCCAGCCTGCAACC CGAAGACTTCGCCACCTACTACTGCCAGCAATGG AGATCCAATCCCCCCACCTTCGGCGGCGGCACCA AGGTTGAGATCAAA CGTACGGTGGCTGCACCATCTGTCTTCATCTTCCC GCCATCTGATGAGCAGTTGAAATCTGGAACTGCC TCTGTTGTGTGCCTGCTGAATAACTTCTATCCCAG AGAGGCCAAAGTACAGTGGAAGGTGGATAACGC CCTCCAATCGGGTAACTCCCAGGAGAGTGTCACA GAGCAGGACAGCAAGGACAGCACCTACAGCCTC AGCAGCACCCTGACGCTGAGCAAAGCAGACTAC GAGAAACACAAAGTCTACGCCTGCGAAGTCACC CATCAGGGCCTGAGTTCGCCCGTCACAAAGAGCT TCAACAGGGGAGAGTGT |
| hz103E9B3H6L5 | Heavy chain | 55 | GAGGTGCAGCTGGTGCAGAGCGGAGCAGAGGTG AAGAAGCCAGGGGCCAGCGTGAAGGTGAGCTGT AAGGCTAGTGGGTACACATTTACAAGCTACACAA TGCACTGGGTGAGACAGGCCCCAGGCCAAGGAC TGGAGTGGATGGGAGACATAAACCCCAATTACGG AGGAACAAGCTACAACGAAAAATTCAAAGACAG AGCCACAATGACCACAGACACCAGCACCAGCAC CGCATACATGGAACTGAGAAGCCTCAGGAGCGAC |
| | | | GACACCGCAGTGTACTACTGCGCCAGAGAGGGA GCCACCGGGACCTGGTACTTCGACGTGTGGGGCC AGGGCACCACCGTGACCGTGAGCAGC GCTAGCACCAAGGGCCCATCGGTCTTCCCCCTGG CACCCTCCTCCAAGAGCACCTCTGGGGGCACAGC GGCCCTGGGCTGCCTGGTCAAGGACTACTTCCCC GAACCGGTGACGGTGTCGTGGAACTCAGGCGCC CTGACCAGCGGCGTGCACACCTTCCCGGCTGTCC TACAGTCCTCAGGACTCTACTCCCTCAGCAGCGT GGTGACCGTGCCCTCCAGCAGCTTGGGCACCCAG ACCTACATCTGCAACGTGAATCACAAGCCCAGCA ACACCAAGGTGGACAAGAAAGTTGAGCCCAAAT CTTGTGACAAAACTCACACATGCCCACCGTGCCC AGCACCTGAACTCCTGGGGGGACCGTCAGTCTTC CTCTTCCCCCCAAAACCCAAGGACACCCTCATGA TCTCCCGGACCCCTGAGGTCACATGCGTGGTGGT GGACGTGAGCCACGAAGACCCTGAGGTCAAGTT CAACTGGTACGTGGACGGCGTGGAGGTGCATAAT GCCAAGACAAAGCCGCGGGAGGAGCAGTACAAC AGCACGTACCGTGTGGTCAGCGTCCTCACCGTCC TGCACCAGGACTGGCTGAATGGCAAGGAGTACA AGTGCAAGGTCTCCAACAAAGCCCTCCCAGCCCC CATCGAGAAAACCATCTCCAAAGCCAAAGGGCA GCCCCGAGAACCACAGGTGTACACCCTGCCCCCA TCCCGGGAGGAGATGACCAAGAACCAGGTCAGC CTGACCTGCCTGGTCAAAGGCTTCTATCCCAGCG ACATCGCCGTGGAGTGGGAGAGCAATGGGCAGC CGGAGAACAACTACAAGACCACGCCTCCCGTGCT GGACTCCGACGGCTCCTTCTTCCTCTACAGCAAG CTCACCGTGGACAAGAGCAGGTGGCAGCAGGGG AACGTCTTCTCATGCTCCGTGATGCATGAGGCTCT GCACAACCACTACACGCAGAAGAGCCTCTCCCTG TCTCCGGGTAAA |
| | Light | 56 | GACATTCAGATGACCCAGAGCCCCAGCAGCCTGA |
| | chain | | GCGCCAGCGTGGGAGACAGAGTGACCATAACCT GCAGCGCCAGCTCCAGCGTGAGCTTCATGTTTTG GTACCAGCAGAAACCCGGCAAGGCCCCCAAGCC CCTGATATACCTGACCAGCAACCTGGCCTCTGGA GTGCCCAGCCGGTTCTCCGGAAGCGGCTCTGGAA CTGACTTCACCCTCACCATCTCCAGCCTGCAACC CGAAGACTTCGCCACCTACTACTGCCAGCAATGG AGATCCAATCCCCCCACCTTCGGCGGCACCA AGGTTGAGATCAAA CGTACGGTGGCTGCACCATCTGTCTTCATCTTCCC GCCATCTGATGAGCAGTTGAAATCTGGAACTGCC TCTGTTGTGTGCCTGCTGAATAACTTCTATCCCAG AGAGGCCAAAGTACAGTGGAAGGTGGATAACGC CCTCCAATCGGGTAACTCCCAGGAGAGTGTCACA GAGCAGGACAGCAAGGACAGCACCTACAGCCTC AGCAGCACCCTGACGCTGAGCAAAGCAGACTAC GAGAAACACAAAGTCTACGCCTGCGAAGTCACC CATCAGGGCCTGAGTTCGCCCGTCACAAAGAGCT TCAACAGGGGAGAGTGT |

## Claims

1. A L1CAM-targeting antibody-drug conjugate, wherein the antibody-drug conjugate comprises an antibody or antigen-binding fragment conjugated to one or more therapeutic agents, wherein the antibody or antigen-binding fragment comprises a light chain variable region having light chain complementarity determining region LCDR1, LCDR2, and LCDR3, and/or a heavy chain variable region having heavy chain complementarity determining region HCDR1, HCDR2, and HCDR3, wherein the heavy chain variable region has a HCDR1, a HCDR2, and a HCDR3 having at least 80%, at least 90%, or 100% identity to the HCDR1, HCDR2, and HCDR3 of the heavy chain variable region sequence selected from the group consisting of SEQ ID NO:21, SEQ ID NO:23, SEQ ID NO:25, SEQ ID NO:27, SEQ ID NO:29, and SEQ ID NO:31, the light chain variable region has a LCDR1, a LCDR2, and a LCDR3 having at least 80%, at least 90%, or 100% identity to the LCDR1, LCDR2 and LCDR3 of the light chain variable region sequence selected from the group consisting of SEQ ID NO:22, SEQ ID NO:24, SEQ ID NO:26, SEQ ID NO:28, SEQ ID NO:30, and SEQ ID NO:32, wherein the therapeutic agent is selected from cytotoxic drugs, microtubule inhibitors, DNA-damaging agents, immunopotentiating agents, and radioactive isotopes, wherein the therapeutic agent is conjugated to the antibody or antigen-binding fragment via a linker.

2. The antibody-drug conjugate of claim 1, wherein the heavy chain variable region and the light chain variable region have a set of CDRs selected from the group consisting of:
(1) a HCDR1, a HCDR2, and a HCDR3 having at least 80%, at least 90%, or 100% identity to the HCDR1, HCDR2, and HCDR3 of the heavy chain variable sequence of SEQ ID NO:21, and a LCDR1, a LCDR2, and a LCDR3 having at least 80%, at least 90%, or 100% identity to the LCDR1, LCDR2, and LCDR3 of the light chain variable region sequence of SEQ ID NO:22;
(2) a HCDR1, a HCDR2, and a HCDR3 having at least 80%, at least 90%, or 100% identity to the HCDR1, HCDR2, and HCDR3 of the heavy chain variable sequence of SEQ ID NO:23, and a LCDR1, a LCDR2, and a LCDR3 having at least 80%, at least 90%, or 100% identity with the LCDR1, LCDR2, and LCDR3 of the light chain variable region sequence of SEQ ID NO:24;
(3) a HCDR1, a HCDR2, and a HCDR3 having at least 80%, at least 90%, or 100% identity to the HCDR1, HCDR2, and HCDR3 of the heavy chain variable sequence of SEQ ID NO:25, and a LCDR1, a LCDR2, and a LCDR3 having at least 80%, at least 90%, or 100% identity to the LCDR1, LCDR2, and LCDR3 of the light chain variable region sequence of SEQ ID NO:26;
(4) a HCDR1, a HCDR2, and a HCDR3 having at least 80%, at least 90%, or 100% identity to the HCDR1, HCDR2, and HCDR3 of the heavy chain variable sequence of SEQ ID NO:27, and a LCDR1, a LCDR2, and a LCDR3 having at least 80%, at least 90%, or 100% identity to the LCDR1, LCDR2, and LCDR3 of the light chain variable region sequence of SEQ ID NO:28;
(5) a HCDR1, a HCDR2, and a HCDR3 having at least 80%, at least 90%, or 100% identity to the HCDR1, HCDR2, and HCDR3 of the heavy chain variable sequence of SEQ ID NO:29, and a LCDR1, a LCDR2, and a LCDR3 having at least 80%, at least 90%, or 100% identity to the LCDR1, LCDR2, and LCDR3 of the light chain variable region sequence of SEQ ID NO:30;
(6) a HCDR1, a HCDR2, and a HCDR3 having at least 80%, at least 90%, or 100% identity to the HCDR1, HCDR2, and HCDR3 of the heavy chain variable sequence of SEQ ID NO:31, and a LCDR1, a LCDR2, and a LCDR3 having at least 80%, at least 90%, or 100% identity to the LCDR1, LCDR2, and LCDR3 of the light chain variable region sequence of SEQ ID NO:32.

3. The antibody-drug conjugate according to claim 1 or 2, wherein:
(1) the HCDR1 has at least 80%, at least 90%, or 100% identity to the sequence selected from: SEQ ID NO:1, SEQ ID NO:7, SEQ ID NO:10, SEQ ID NO: 16;
(2) the HCDR2 has at least 80%, at least 90%, or 100% sequence identity to the sequence selected from: SEQ ID NO:2, SEQ ID NO:8, SEQ ID NO: 11, SEQ ID NO: 17;
(3) the HCDR3 has at least 80%, at least 90%, or 100% identity to the sequence selected from: SEQ ID NO:3, SEQ ID NO:9, SEQ ID NO:12, SEQ ID NO:18;
(4) the LCDR1 has at least 80%, at least 90%, or 100% identity to the sequence selected from: SEQ ID NO:4, SEQ ID NO:13;
(5) the LCDR2 has at least 80%, at least 90%, or 100% identity to the sequence selected from: SEQ ID NO:5, SEQ ID NO: 14, SEQ ID NO:19;
(6) the LCDR3 has at least 80%, at least 90%, or 100% identity to the sequence selected from: SEQ ID NO:6, SEQ ID NO: 15, SEQ ID NO:20.

4. The antibody-drug conjugate according to any one of claims 1 to 3, wherein:
(1) the HCDR1 comprises the amino acid sequence as set forth in SEQ ID NO: 1;
(2) the HCDR2 comprises the amino acid sequence as set forth in SEQ ID NO:2;
(3) the HCDR3 comprises the amino acid sequence as set forth in SEQ ID NO:3;
(4) the LCDR1 comprises the amino acid sequence as set forth in SEQ ID NO:4;
(5) the LCDR2 comprises the amino acid sequence as set forth in SEQ ID NO:5;
(6) the LCDR3 comprises the amino acid sequence as set forth in SEQ ID NO:6.

5. The antibody-drug conjugate according to any one of claims 1 to 3, wherein:
(1) the HCDR1 comprises the amino acid sequence as set forth in SEQ ID NO:7;
(2) the HCDR2 comprises the amino acid sequence as set forth in SEQ ID NO:8;
(3) the HCDR3 comprises the amino acid sequence as set forth in SEQ ID NO:9;
(4) the LCDR1 comprises the amino acid sequence as set forth in SEQ ID NO:4;
(5) the LCDR2 comprises the amino acid sequence as set forth in SEQ ID NO:5;
(6) the LCDR3 comprises the amino acid sequence as set forth in SEQ ID NO:6.

6. The antibody-drug conjugate according to any one of claims 1 to 3, wherein:
(1) the HCDR1 comprises the amino acid sequence as set forth in SEQ ID NO: 10;
(2) the HCDR2 comprises the amino acid sequence as set forth in SEQ ID NO: 11;
(3) the HCDR3 comprises the amino acid sequence as set forth in SEQ ID NO: 12;
(4) the LCDR1 comprises the amino acid sequence as set forth in SEQ ID NO:13;
(5) the LCDR2 comprises the amino acid sequence as set forth in SEQ ID NO:14;
(6) the LCDR3 comprises the amino acid sequence as set forth in SEQ ID NO:15.

7. The antibody-drug conjugate according to any one of claims 1 to 3, wherein:
(1) the HCDR1 comprises the amino acid sequence as set forth in SEQ ID NO:16;
(2) the HCDR2 comprises the amino acid sequence as set forth in SEQ ID NO: 17;
(3) the HCDR3 comprises the amino acid sequence as set forth in SEQ ID NO: 18;
(4) the LCDR1 comprises the amino acid sequence as set forth in SEQ ID NO:13;
(5) the LCDR2 comprises the amino acid sequence as set forth in SEQ ID NO:19;
(6) the LCDR3 comprises the amino acid sequence as set forth in SEQ ID NO:20.

8. The antibody-drug conjugate according to any one of claims 1 to 3, wherein the heavy chain variable region comprises an amino acid sequence of any one selected from the group consisting of:
(1) an amino acid sequence having at least 90%, at least 95%, or 100% sequence identity with SEQ ID NO:21;
(2) an amino acid sequence having at least 90%, at least 95%, or 100% sequence identity with SEQ ID NO:23;
(3) an amino acid sequence having at least 90%, at least 95%, or 100% sequence identity with SEQ ID NO:25;
(4) an amino acid sequence having at least 90%, at least 95%, or 100% sequence identity with SEQ ID NO:27;
(5) an amino acid sequence having at least 90%, at least 95%, or 100% sequence identity with SEQ ID NO:29;
(6) an amino acid sequence having at least 90%, at least 95%, or 100% sequence identity with SEQ ID NO:31.

9. The antibody-drug conjugate according to any one of claims 1 to 3, wherein the light chain variable region comprises an amino acid sequence of any one selected from the group consisting of:
(1) an amino acid sequence having at least 90%, at least 95%, or 100% sequence identity with SEQ ID NO:22;
(2) an amino acid sequence having at least 90%, at least 95%, or 100% sequence identity with SEQ ID NO:24;
(3) an amino acid sequence having at least 90%, at least 95%, or 100% sequence identity with SEQ ID NO:26;
(4) an amino acid sequence having at least 90%, at least 95%, or 100% sequence identity with SEQ ID NO:28;
(5) an amino acid sequence having at least 90%, at least 95%, or 100% sequence identity with SEQ ID NO:30;
(6) an amino acid sequence having at least 90%, at least 95%, or 100% sequence identity with SEQ ID NO:32.

10. The antibody-drug conjugate according to any one of claims 1 to 9, wherein the antibody is a monoclonal antibody, a bispecific antibody, a multispecific antibody, a recombinant antibody, a chimeric antibody, a bivalent antibody, an anti-idiotype antibody, or a fusion protein.

11. The antibody-drug conjugate according to any one of claims 1 to 9, wherein the linker is linked to the antibody via a thiol group.

12. The antibody-drug conjugate according to any one of claims 1 to 10, wherein the therapeutic agent is selected from any one of auristatin cytotoxic molecules (e.g., MMAE, MMAF), maytansinoid cytotoxic molecules (e.g., DM1, DM4), ansamycins derivatives PBD, camptothecins and camptothecin derivatives (e.g., Exatecan, Dxd) or any combination thereof.

13. An antibody-drug conjugate having the structure of formula (I) as following: 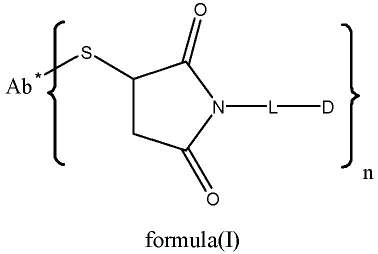
wherein "Ab" refers to an antibody or antigen-binding fragment selected from the antibody or antigen-binding fragment of any of claims 1 to 9;
wherein, L refers to a cleavable or non-cleavable linker;
wherein, D refers to a therapeutic agent;
wherein, n refers to the drug-to-antibody ratio (DAR), preferably ranging from 1 to 8.

14. The antibody-drug conjugate according to claim 13, wherein L refers to a cleavable linker.

15. The antibody-drug conjugate according to claim 13 or 14, wherein L refers to -L₁-L₂-L₃-, wherein L₁ refers to any one of: -(CH₂)ₙ-X-Y-(CH₂)ₘ-, -X (CH₂)ₙ-O-(CH₂CH₂O)ₚ-(CH₂)ₘ-Y-, -(CH₂)ₙ-X-, -X- (CH₂)ₘ-Y-, or any combination thereof, wherein X and Y each independently refers to -C(O)-, O, -CR¹R²-, -NR¹-, S, or is not present, wherein R¹ and R² each independently refers to hydrogen, C₁-C₆ alkyl, or C₃-C₆ cycloalkyl, wherein m, n, and p each independently refers to an integer from 0 to 10;
wherein L₂ refers to an amino acid residue;
wherein L₃ is not present or refers to, -C(O)-, or -PAB-C(O);
wherein PAB refers to p-aminobenzyloxy.

16. The antibody-drug conjugate according to claim 15, wherein L₂ refers to any one of -Val-Cit-, -Val-Ala-, -Gly-Gly-Phe-Gly-, Ala-Ala-Asn-, -Val-Lys, -Phe-Lys, -Phe-Cit, -Phe-Arg-, -Phe-Ala-, -Ala-Lys, -Leu-Cit-, -Ile-Cit-, -Trp-Cit-, -D-Phe-LPhe-Lys-, -Phe-Phe-Lys-, -D-Phe-Phe-Lys-, -Gly-Phe-Lys-, -Gly-Phe-Leu-Gly-, Ala-Leu-Ala-Leu or any combination thereof.

17. The antibody-drug conjugate according to claim 16, wherein L refers to -(CH₂)₅-Val-Cit-PAB-C(O)- or -(CH₂)₅-Val-Ala-PAB-C(O)-.

18. The antibody-drug conjugate according to claim 17, wherein the therapeutic agent selected from any one of MMAE, MMAF, Dxd, DM1, DM4 or a combination thereof.

19. An antibody-drug conjugate having the following structure: 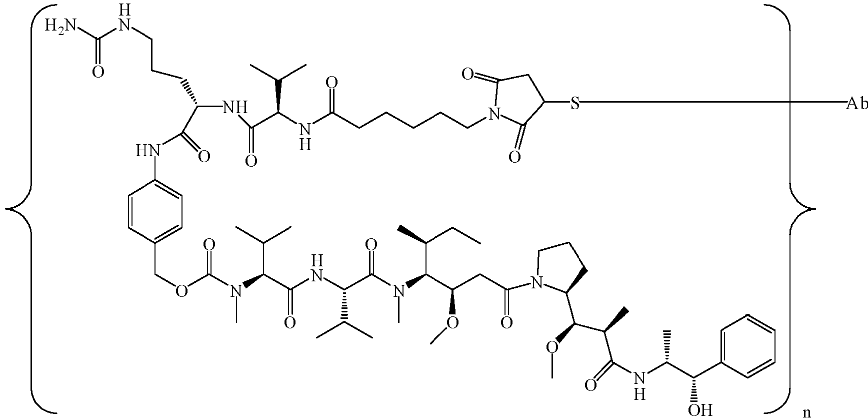 or 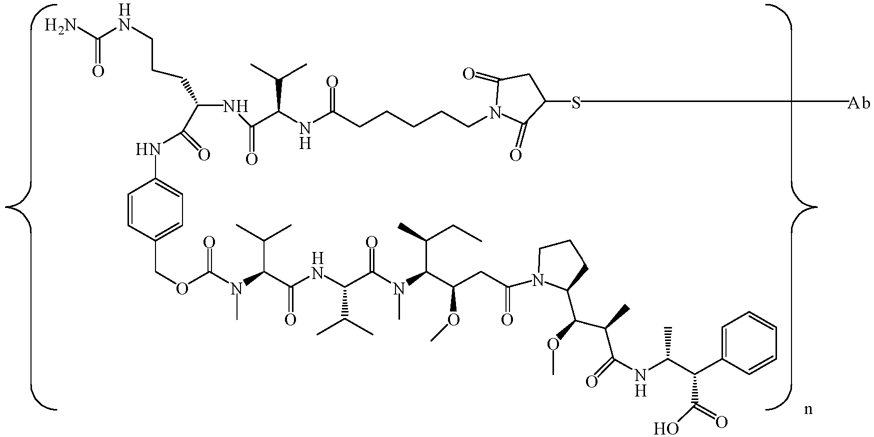 wherein "Ab" refers to an antibody or antigen-binding fragment selected from the antibody or antigen-binding fragment of any of claims 1 to 9, wherein n refers to the drug-to-antibody ratio (DAR), preferably ranging from 1 to 8.

20. A pharmaceutical composition comprising the antibody-drug conjugate of any of claims 1 to 19 and a pharmaceutically acceptable carrier.

21. Use of the antibody-drug conjugate of any of claims 1 to 19 in the manufacture of a medicament for the treatment of a L1CAM-related cancer.

22. A method for treating a L1CAM-related cancer, comprising administering the antibody-drug conjugate of any of claims 1 to 19 to a subject having the cancer.
